(19) Europäisches Patentamt

European Patent Office

Office européen des brevets

(11) **EP 0 640 216 B1**

(12) **EUROPEAN PATENT SPECIFICATION**

(45) Date of publication and mention
of the grant of the patent:
**02.10.2002 Bulletin 2002/40**

(21) Application number: **92907150.4**

(22) Date of filing: **20.03.1992**

(51) Int Cl.7: **G01N 33/537**, G01N 33/543,
G01N 27/327, G01N 33/50,
G01N 33/58, G01N 33/18,
G01N 33/24, G01N 33/04

(86) International application number:
**PCT/GB92/00506**

(87) International publication number:
**WO 92/016838 (01.10.1992 Gazette 1992/25)**

(54) **SEPARATION METHOD**

ABTRENNUNGSVERFAHREN

TECHNIQUE DE SEPARATION

(84) Designated Contracting States:
**AT CH DE FR IT LI NL**

(30) Priority: **20.03.1991 GB 9105921**
**24.12.1991 GB 9127346**

(43) Date of publication of application:
**01.03.1995 Bulletin 1995/09**

(73) Proprietor: **Marconi Optical Components Limited**
**London WIX 8AQ (GB)**

(72) Inventors:
• **ABUKNESHA, Ramadan Arbi**
**Fortune Green Road, London NW6 1DP (GB)**
• **BYFIELD, Mark Philip**
**Slough, Berkshire SL2 5QW (GB)**

(74) Representative: **Cockayne, Gillian et al**
**Marconi Intellectual Property**
**Marrable House**
**The Vineyards**
**Gt. Baddow**
**Chelmsford Essex CM2 7QS (GB)**

(56) References cited:
**EP-A- 0 167 248**     **EP-A- 0 177 191**
**EP-A- 0 188 093**     **EP-A- 0 282 192**
**EP-A- 0 363 510**     **EP-A- 0 416 730**
**WO-A-86/01407**     **WO-A-88/02776**
**WO-A-90/01167**     **FR-A- 2 476 125**

• **CLINICAL CHEMISTRY. vol. 30, no. 9, September
1984, WINSTON US pages 1457 - 1461; S. J.
RATTLE ET AL.: 'New separation method for
monoclonal immunoradiometric assays and its
application to assays for Thyrotropin and
Human Choriogonadotropin.'**
• **JOURNAL OF THE AMERICAN CHEMICAL
SOCIETY. vol. 104, no. 24, 1982, GASTON, PA US
pages 6733 - 6737; R. S. REID ET AL.: 'Nuclear
magnetic resonance studies of the solution
chemistry of metal complexes. 19. Formation
constants for the complexation of
methylmercury by glutathione, ergothioneine,
and hemoglobin.'**

**Description**

The present invention relates to a separation method which finds application in the detection of species (e. g. in immunoassay and in immunosensors), and to a method suitable for use in detection, to the use a sensor in the method of detection in accordance with the present invention, and to the use of a test kit in a method in accordance with the present invention.

According to one aspect of the present invention there is provided a separation method, suitable for use in an immunoassay method for the detection of an analyte species, which separation method includes arranging for an auxiliary species and a binding species for the auxiliary species to undergo specific binding, said auxiliary species not being a primary species and said auxiliary species being a ligand and said auxiliary species being provided on a support material, said support material being substantially non-permeable, said binding species being an antibody capable of specific binding with the auxiliary species and said binding species being capable of being linked with a primary species by means of a linkage which involves a link, of a non-specific binding type, to the binding species, said binding species being arranged to be linked with a primary species by means of said linkage.

An example of a link of a non-specific binding type is a covalent link. Thus, for example, the linkage may involve a covalent link to the binding species.

Another example of a link of a non-specific binding type is a link which involves adsorption. Thus, for example, a binding species and a primary species may be linked by both being adsorbed on a suitable material (e.g. a carrier material such as latex particles).

The binding species may be linked with the primary species directly or indirectly (e.g. via other species).

Thus, for example, where the binding species is arranged to be linked directly to a primary species the linkage may be a link of a non-specific binding type (e.g. a covalent link) between the binding species and the primary species.

By way of further example, where the binding species is arranged to be linked indirectly to a primary species the linkage may include one or more other species, and one or more links, as required, at least one of which links being a link, of a non-specific binding type, to the binding species.

It is to be understood that where one or more links are involved in the linkage, one of the links will be a link of a non-specific binding type and any further link or links in the linkage may be of any suitable type (e.g. non-specific binding type or specific linking type); thus, for example, the linkage may include a further link or links of a non-specific binding type, or may include a further link or links of a specific binding type (e.g. ligand-binder type), or a mixture of types of links (e.g. non-specific binding type and specific binding type).

Where the binding species is linked to a primary species via other species, said other species may be, for example, a second antibody or a ligand or a binder and, for example, the binding species may be covalently linked to said other species.

It is to be understood that separation in accordance with the present invention may be effected by arranging for the auxiliary species and the binding species to bind together.

According to further aspect of the present invention there is provided a method, suitable for use in the detection of an analyte species by immunoassay, which method includes arranging for an auxiliary species and a binding species for the auxiliary species to undergo specific binding, said auxiliary species not being a primary species and said auxiliary species being a ligand and said auxiliary species being provided on a support material, said support material being substantially non-permeable, said binding species being an antibody capable of specific binding with the auxiliary species and said binding species being capable of being linked with a primary species by means of a linkage which involves a link, of a non-specific binding type, to the binding species, said binding species being arranged to be linked with a primary species by means of said linkage.

By way of example, in accordance with the present invention, an analyte species may be detected as such, or an analyte species may be part of an entity to be detected (e.g. the analyte species may be part of an entity which is an analyte species-containing entity). Also, by way of example, the present invention may utilise an antibody to an analyte species or an antibody to an entity to be detected.

In accordance with the present invention there is also provided a separation method, suitable for use in an immunoassay method for the detection of an analyte species, which separation method includes the use of an auxiliary species, being a ligand, provided on a support material, the use of a binding species for the auxiliary species said binding species being an antibody capable of specific binding with the auxiliary species and said binding species being capable of being linked with a primary species by means of a linkage which involves a link, of a non-specific binding type, to the binding species, and the use of an antibody to an analyte species or the use of an antibody to an entity to be detected.

An antibody to an analyte species, or an antibody to an entity to be detected, may be prepared by any suitable method, for example those known for the raising of polyclonal or monoclonal antibodies; thus, antibodies may be raised, for example, by immunising animals.

In accordance with an embodiment of the present invention there is provided a separation method, suitable

EP 0 640 216 B1

for use in an immunoassay method for the detection of an analyte species, which separation method includes the use of an auxiliary species, being a ligand, provided on a support material, the use of a binding species for the auxiliary species said binding species being an antibody capable of specific binding with the auxiliary species and said binding species carrying an entity to be detected, and the use of an antibody to the entity to be detected.

The present invention also provides a method, suitable for use in the detection of an analyte species by immunoassay, which method includes the use of an auxiliary species, being a ligand, provided on a support material, the use of a binding species for the auxiliary species said binding species being an antibody capable of specific binding with the auxiliary species and said binding species being capable of being linked with a primary species by means of a linkage which involves a link, of a non-specific binding type, to the binding species, and the use of an antibody to an analyte species or the use of an antibody to an entity to be detected.

In accordance with an embodiment of the present invention there is provided a method, suitable for use in the detection of an analyte species by immunoassay, which method includes the use of an auxiliary species, being a ligand, provided on a support material, the use of a binding species for the auxiliary species, said binding species being an antibody capable of specific binding with the auxiliary species and said binding species carrying an entity to be detected, and the use of an antibody to the entity to be detected.

According to another aspect of the present invention there is provided use of a sensor, which sensor includes a support material and an auxiliary species provided on said support material, in a method in accordance with the present invention.

By way of example, a sensor used in accordance with the present invention may include a surface suitable for acting as a support material for an auxiliary species.

In accordance with an embodiment of the present invention a sensor used in accordance with the present invention may include a support material having a surface upon which there is immobilised the auxiliary species.

The surface may be, for example, glass, quartz or an electrode material.

According to a further aspect of the present invention there is provided use of a support material, wherein an auxiliary species is provided on said support material, in a separation method in accordance with the present invention.

According to a further aspect of the present invention there is provided use of a support material wherein an auxiliary species is provided on said support material, in a method in accordance with the present invention.

The binding species may be arranged to carry the entity to be detected in any suitable manner and at any suitable time. Thus, the binding species may, for example, be arranged to carry the entity either before or after the binding between the auxiliary species and the binding species has taken place.

A primary species in accordance with the present invention is a species capable of taking part in a primary immune binding reaction. By way of example, a primary immune binding reaction in accordance with the present invention is one in which an analyte species undergoes, or an entity to be detected (containing an analyte species) undergoes, a specific binding reaction or an authentic analyte species (as hereinafter defined) undergoes, or an authentic entity to be detected (as hereinafter defined) undergoes, a specific binding reaction or an analyte species and an authentic analyte species undergo specific binding reactions or an entity to be detected and an authentic entity to be detected undergo specific binding reactions. (It will be appreciated that the analyte species and the authentic analyte species undergo specific binding reactions with other species and not with each other and that the entity to be detected and the authentic entity to be detected undergo specific binding reactions with other species and not with each other.)

By way of example, a primary species may be a primary antibody or a ligand (e.g. an antigen). It is to be understood that, for example, a primary species may be an antibody to an analyte species, an antibody to an authentic analyte species, an antibody to an entity to be detected or an antibody to an authentic entity to be detected; it will be appreciated that, for a given immunoassay, the antibody to the analyte species and the antibody to the authentic analyte species will be the same antibody; similarly, it will be appreciated that, for a given immunoassay, the antibody to the entity to be detected and the antibody to the authentic entity to be detected will be the same antibody. It is also to be understood that a primary species may be, for example, an analyte species, an authentic analyte species, an entity to be detected or an authentic entity to be detected.

The term "antibody" as used in this Specification (e.g. in relation to a primary antibody or a second antibody) embraces whole antibody or antibody fragments such as Fab and $(Fab)_2$ and, accordingly, the term "antibodies" used herein embraces whole antibodies and antibody fragments.

Also it is to be understood that an "authentic analyte species" is a species which is capable of reacting in a substantially similar manner as an analyte species to be detected under substantially similar conditions; similarly, it is to be understood that an "authentic entity to be detected" is an entity which is capable of reacting in a substantially similar manner as an entity to be detected under substantially similar conditions.

It will be appreciated that an authentic analyte species may be used, inter alia, as a calibrator or a standard; similarly, it will be appreciated that an authentic entity to be detected may be used, *inter alia*, as a calibrator or a standard.

The auxiliary species is a species which does not itself take part in a primary specific binding immune reaction with an analyte species (or an authentic analyte species) or an entity to be detected (or an authentic entity to be

3

detected).

The auxiliary species may be, for example, provided on a support material in any suitable manner, and it is to be understood that in this Specification "providing on a support material" and "provided on a support material" embrace, for example, situations where the support material itself, or a part of the support material itself, provides auxiliary species and situations where the support material carries an auxiliary species.

Thus, for example, the auxiliary species may be provided by chemical groups or units of the support material or the auxiliary species may be, for example, attached to the support material (e.g. by covalent linkage or adsorption). Where the support material is, for example, a polymer, units of the polymer may act as auxiliary species. Also, by way of example, surface groups present on a support material, such as polystyrene or modified silica, may act as auxiliary species.

By way of example, instead of arranging for the support material to provide single auxiliary species, the support material may, if desired, provide oligomers or polymers of auxiliary species.

Where an auxiliary species is attached to a support material the auxiliary species may be directly attached to the support material or indirectly attached to the support material via other species (e.g. a carrier protein).

In one embodiment the present invention provides a method which also includes the step of attaching, either directly, or indirectly, an auxiliary species to a support material.

By way of further example, the surface of a support material may be activated thereby to permit attachment of ligands; for example, the surface of a suitable support material may be activated by chemical treatment to provide free amino groups to which ligands may be linked.

Further, by way of example, instead of linking single auxiliary species to free amino groups, (produced as immediately hereinbefore disclosed) oligomers of auxiliary species or polymers of auxiliary species may be attached to the free amino groups.

Also, by way of example, an auxiliary species may be linked (e.g. covalently or otherwise) to a further species (e.g. a carrier protein or a polymer) and the further species may be associated with the support material such that the auxiliary species may become indirectly provided on the support material.

By way of further example, instead of linking single auxiliary species indirectly to a support material it is possible to link (e.g. covalently or otherwise) oligomers or polymers of auxiliary species to a further species (e.g. a carrier protein or a polymer) and the further species may be associated with the support material such that oligomers of auxiliary species or polymers of auxiliary species may become indirectly provided on the support material.

Where an auxiliary species is attached to a support material, attachment of the auxiliary species may be effected at any desired time. Thus, for example, the auxiliary species may be allowed to bind with the binding species before, or after, the auxiliary species is attached to the support material; however, in general, it may be more convenient to attach the auxiliary species to the support material before bringing together the auxiliary species and the binding species.

It is to be understood that the use of oligomers or polymers of auxiliary species may be advantageous in certain circumstances. Thus, for example, by use of an oligomer or a polymer of an auxiliary species more auxiliary species may be provided than it is possible to provide when using only single auxiliary species. Accordingly, for example, the provision of more auxiliary species offers the possibility of faster reactions since more auxiliary species are available to undergo reaction with available binding species therefor.

The binding species may be arranged to bind with the auxiliary species in any suitable manner and at any suitable time. Thus, for example, the binding species may be arranged to be linked (either directly or indirectly) with a primary species either before, or after, binding with the auxiliary species.

The auxiliary species may be a suitable ligand examples of which are antigenic ligands (such as haptens).

Examples of antigenic ligands are 2,4 dinitrophenol, fluorescein, digitoxin, coumarin and cibacron blue.

Further examples of auxiliary species which may be attached to a support material (e.g. by covalent linkage or non-covalent attachment (adsorption)) are soluble materials for example soluble molecules (e.g. polymers) such as polypeptides, proteins, polysaccharides and conducting polymers; haptenic functions may be covalently coupled to such soluble materials (e.g. polymers). By way of example, an auxiliary species may be provided as a coating of a polymer on a support material.

The binding species may be any suitable antibody capable of binding with the auxiliary species; the binding species may therefore be considered to be an "auxiliary" binding species in that it is capable of binding with the auxiliary species.

It will be appreciated that an antibody may be regarded as a binding protein.

Where, for example, the auxiliary species is an antigenic ligand, the binding species may be an antibody to an antigenic ligand. An antibody to the auxiliary species may be considered to be an anti-auxiliary species antibody.

Accordingly, where, for example, the auxiliary species is fluorescein, coumarin, 2,4 dinitrophenol or cibacron blue, the binding species may be, respectively, anti-fluorescein antibody, anti-coumarin antibody, anti-2,4 dinitrophenol antibody or anti-cibacron blue antibody.

It will be appreciated that antibodies such as those immediately hereinbefore disclosed may be prepared by any suitable method, for example those known for the raising of polyclonal or monoclonal antibodies; thus, antibodies may be raised, for example, by immunising animals with conjugates made of suitable derivatives of a ligand and an immunogenic carrier protein such as bovine serum albumin or key-hole limpet haemocyanin; the product obtained by immunising animals may be purified as desired (e.g. by the use of affinity chromatography) to obtain the required antibodies.

The separation method of the present invention may, therefore, for example, utilise separation systems comprising fluorescein/anti-fluorescein antibody, coumarin/anti-coumarin antibody, 2,4 dinitrophenol/anti-2,4 dinitrophenol antibody, or cibacron blue/anti-cibacron blue antibody.

The present invention may find application, for example, in the detection of an analyte species, or in the detection of an entity to be detected, in any suitable sample. Thus, for example, samples of water, soil, living species (such as plants (e.g. vegetables) or animals) or air may provide an analyte species, or an entity to be detected, for detection in accordance with the present invention. Examples of biological samples in which an analyte species, or an entity to be detected, may be detected in accordance with the present invention are blood, plasma, serum, urine, saliva and milk. An analyte species, or an entity to be detected, may be, for example, present in water, an aqueous preparation or a fluid extract. An analyte species may be, for example, a hapten.

Examples of analyte species which may be detected in accordance with the present invention are:

(a) steroid hormones such as progesterone, $17\alpha$-hydroxy progesterone or estradiol (e.g. in a sample of blood, serum, saliva, urine or milk),
(b) hormones such as a thyroid hormone (e.g. thyroxine or triiodothyronine),
(c) drugs such as drugs of abuse (e.g. phenobarbital) and therapeutic drugs (e.g. digoxin) (in for example, a sample of blood, serum, saliva or urine),
(d) polypeptide hormones (e.g. in a sample of blood or urine),
(e) tumour markers such as marker proteins (e.g. in a sample of blood or serum),
(f) human serum albumin,
(g) human chlorionic gonadotrophin (hCG),
(h) human IgG,
(i) protein antigens,
(j) blood proteins (e.g. immunoglobulins, enzyme markers or receptors),
(k) marker proteins resulting from urine disease tests,
(l) insecticides, pesticides, or herbicides (e.g. in water or soil),
(m) toxins (such as those extracted from feeds and food stuffs), and
(n) micro-organisms (e.g. viruses and bacteria).

Further examples of analyte species which may be detected in accordance with the present invention are complexes of metals.

Thus, the present invention may find application in, for example, the detection of complexes of metals such as strong metal complexes which may be regarded as toxic (e.g. in biological terms when present in the environment).

An example of a metal complex which may be detected in accordance with the present invention is methyl mercury.

By way of further example, metal ions may be formed in to a complex of a metal by use of a complexing agent (e.g. a chelating agent) and the complex thus formed may act as an analyte species in accordance with the present invention.

Also, by way of further example, metals may be detected in accordance with the present invention as is further disclosed hereinafter.

Examples of support materials which may find application in accordance with the present invention are solid phase materials such as a reaction vessel wall, insoluble polysaccharides, microparticles (e.g. particulate microcellulose), polystyrene (e.g. in the form of wells, beads, microtitre plates, discs, sticks or tubes), cross-linked dextran (e.g. Sephadex), insoluble polymer structures, glass surfaces, derivatised silica surfaces (e.g. having silyl groups with chemical functions attached), soluble polymers attached to a suitable surface (e.g. a glass surface), microparticulate materials with entrapped ferrous oxide (magnetisable particles), nylon and polyamides.

It will be appreciated that some types of support materials may be inappropriate for use with some analyte species and some entities to be detected. Thus, for example, where the analyte species is a metal ion, or the entity to be detected contains a metal, the use of some types of support materials may be inappropriate (e.g. support materials containing entrapped iron oxide may give rise to unacceptable interference).

The present invention may find application in, for example, label-free or detectable species-dependent (e.g. tracer species-dependent) assay methods such as enzyme-immunoassay, fluoro-immunoassay and radio-immunoassay. By way of example, the present invention may find application in antibody-labelled or antigen-labelled assays.

By way of example, any suitable detectable species may be used in accordance with the present invention in the detection of an analyte species or an entity to be detected. It is to be understood that a detectable species may be, for example, a detectable structure.

Examples of detectable species are enzymes, fluorophores (or polymeric fluorophores), radioisotopes, ligands (or polymers of a ligand), and binders.

By way of example, an enzyme may be detected by a corresponding substrate, fluorophores and radioisotopes may be detected directly with suitable detectors, ligands may be detected by use of binders therefor, said binders being associated with tracer species, and binders may be detected by use of ligands therefor, said ligands being associated with tracer species.

The tracer species may be, for example, any suitable tracer species such as those known in the art relating to protein binding assays (e.g. immunoassays). (It is to be understood that a tracer species may also be considered to be a signal species or a labelling species.)

Examples of tracer species are enzymes (e.g. alkaline phosphatase, β-galactosidase and horse-radish peroxidase), fluorophores (e.g. fluoresceins, coumarins or rhodamin), chemiluminescent compounds, bioluminescent compounds, radioisotopes and dyes.

Detection or measurement of a signal from a detectable species may be carried out in any suitable manner such as those known in the immunochemical field.

Where, for example, a ligand or a binder is used (as hereinbefore disclosed) as a detectable species any suitable ligand or binder may be utilised.

Examples of ligands which may be utilised as detectable species are antigens (e.g. 2,4 dinitrophenol, fluorescein, digitoxin, coumarin and cibacron blue), which may be considered to be haptens, and non-antigenic ligands such as the ligand of a specific ligand-binder pair (e.g. biotin).

Examples of binders which may be utilised as detectable species are antibodies (e.g. anti-2,4 dinitrophenol antibody, anti-fluorescein antibody, anti-digitoxin antibody, anti-coumarin antibody and anti-cibacron blue antibody) and binders of a specific ligand-binder pair (e.g. avidin). It will be appreciated that antibodies such as those immediately hereinbefore disclosed may be prepared by any suitable method such as those known for the raising of polyclonal or monoclonal antibodies; thus antibodies may be raised, for example, by immunising animals.

The present invention may find application in any suitable form of immunological detection or immunoassay examples of which are competitive-immunoassay methods, non-competitive immunoassay methods and sandwich immunoassay methods.

Where a detectable species is used in accordance with the present invention, the detectable species may be, for example, arranged to be associated, in any suitable manner and at any suitable time, with any chosen species or entity; thus, for example, a detectable species may be associated (as appropriate to an immunoassay being utilised) with a binding species, or with an authentic analyte species, or with an authentic entity species (being an authentic entity to be detected), or with a primary antibody, or with a second antibody.

The present invention will now be further described, by way of example only, with reference to a competitive immunoassay method and with reference to a non-competitive immunoassay method.

In the two immunoassay methods described immediately hereinafter, use is made of an anti-auxiliary species antibody. (Where, for example, the auxiliary species is a hapten which is antigenic, the anti-auxiliary species antibody may be considered to be an anti-auxiliary hapten antibody.) As hereinbefore disclosed anti-auxiliary species antibodies may be prepared by any suitable method, for example those known for the raising of polyclonal and monoclonal antibodies.

By way of example, in a competitive immunoassay method which includes a separation method in accordance with the present invention the following may take place:

(a) an auxiliary species is introduced in high concentration to a support material (e.g. by covalent linkage) or a support material itself is arrange to provide auxiliary species;

(b) a previously prepared antibody to the auxiliary species is linked, by covalent linking, to an authentic analyte species of interest;

(c) an anti-analyte antibody (which may be termed, for example, a primary antibody) is labelled with a tracer species (capable of emitting a signal) (e.g. a fluorescent substance or an enzyme);

(d) in what may be termed the primary immune reaction, analyte species (if present), suitable amounts of labelled primary antibody, and authentic analyte species linked to the antibody to the auxiliary species are allowed to mix in solution.

The primary immune reaction may be expected to attain or approach equilibrium in a short period of time (e.g. at a temperature of 37°C to 42°C) thereby to produce a primary immune reaction mixture. It will be appreciated that the primary reaction mixture may contain, *inter alia*, bound and unbound fractions.

Subsequently a moderate "excess" of auxiliary species provided on a support material (e.g. either carried by the support material or provided by the support material itself or a part of the support material itself) is added to the primary immune reaction mixture or the primary immune reaction mixture is brought into contact with an "excess" of auxiliary species provided on a support material (e.g. provided by the support material, or part thereof, or attached thereto).

(It will be appreciated that "excess" as used in the immediately preceding paragraph means an "excess" in terms of auxiliary species sites with respect to the corresponding binding species to the auxiliary species.)

Because the auxiliary species is present in very large excess with respect to the antibody to the auxiliary species, the binding reaction may be expected to occur rapidly and efficiently with a high proportion of the antibody to the auxiliary species becoming bound to the support material. By way of example, in some circumstances, but not all, as much as 99% of the antibody may become bound to the support material.

Thus, by means of the binding between the binding species and the auxiliary species, some of the antibody carrying the tracer species may be retained on the support material by virtue of being linked to the authentic analyte species which, in turn, is linked to the binding species. The proportion of retained antibody carrying the tracer species will depend upon the concentration of "competing" analyte species, if any, in a given sample; the competing analyte species may be a standard or an unknown quantity.

Washing of the support material with appropriate buffers may be used to remove unbound materials and tracer species activity associated with the support material may be measured by any suitable method such as those known in the art.

Optionally, tracer species activity may be eluted into solution using appropriate buffers to facilitate measurement of tracer species.

In some embodiments of the present invention (e.g. embodiments involving the use of an optical immunosensor based on the surface plasmon resonance principle or on evanescent wave fluorescence) a washing step may not be required; thus, tracer activity associated with the support material (which may be, for example, a sensor surface) may be measured in the presence of unbound material.

It will be understood that, by use of standard quantities of authentic analyte species, calibration may be effected such that, subsequently, unknown quantities of analyte species may be determined. It will also be appreciated that, in a competitive immunoassay, as the amount of analyte species increases, the amount of tracer species retained on the support material decreases and, conversely, as the amount of analyte species decreases the amount of tracer species retained on the support material increases, reaching a maximum when no analyte species is present.

By way of example, in a non-competitive immunoassay method which involves a separation method in accordance with the present invention the following may be utilised:

(a) an antibody to the analyte species (a "signal" antibody) which is conjugated to a tracer species which is capable of emitting a signal (e.g. a fluorescent substance or an enzyme);
(b) a hybrid complex comprising an "immobilising" antibody which is conjugated to an antibody to an auxiliary species;
(c) an analyte species (which may be, for example, an antigenic substance of high molecular weight);
(d) a support material which itself provides or has attached thereto an auxiliary species.

Also, by way of example, in a non-competitive immunoassay method which includes a separation method in accordance with the present invention the following may take place:

(i) an analyte species of interest and the "signal" antibody conjugated to the tracer species are mixed in solution whereupon analyte species is bound by the signal antibody;
(ii) to the reaction mixture formed in (i) in solution a moderate "excess" of immobilising antibody hybrid complex (i.e. immobilising antibody conjugated to an antibody to the auxiliary species) is added whereupon an immune complex "sandwiching" the analyte species is formed;
(iii) excess support material (e.g. which itself provides or has attached thereto an auxiliary species) is added to the reaction mixture formed in (ii), or the reaction mixture formed in (ii) is brought into contact with an excess of support material (e.g. which itself provides or has attached thereto an auxiliary species), such that binding of the antibody to the auxiliary species (which antibody is conjugated to the immobilising antibody) to the auxiliary species (supplied by or on the support material) takes place. By virtue of this binding, only "signal" antibody bound to the analyte species becomes attached to the support material.

washing the support material with appropriate buffer may be used to remove unattached "signal" antibody.

The tracer species left on the support material, the amount of which is directly proportional to the concentration of the analyte species, may be measured by any suitable method such as those known in the art.

In some embodiments of the present invention (e.g. embodiments involving the use of an optical immunosensor based on the surface plasmon resonance principle or on evanescent wave fluorescence) a washing step may not be required; thus, tracer activity associated with the support material (which may be, for example, a sensor surface) may be measured in the presence of unbound material.

It will be understood that by use of standard quantities of authentic analyte species calibration may be effected such that, subsequently, unknown quantities of analyte species may be determined.

It will be appreciated that in heterogenous immunochemical analysis (immunoassay) there is a requirement for the separation of bound and unbound fractions of detectable species (e.g. a tracer species) in order to permit assessment of the distribution of detectable species (e.g. tracer species) between bound and unbound states. It may be considered that this separation step in an immunoassay method is one of the most important features of such a method.

The practical importance of a separation step is based upon the fact that the efficiency of the step, the simplicity (or otherwise) thereof, and the speed thereof may influence the general properties of an assay method and may influence the performance of an assay method.

Separation of bound and unbound fractions of detectable species (e.g. tracer species) may be effected using a solid phase immobilised antibody technique in which antibody is adsorbed onto a surface of a reaction vessel (e.g. polystyrene tubes) or covalently linked to a micro-particulate material such as microcellulose, Sephadex or micro-particulate material with entrapped ferrous oxide (magnetisable particles).

The use of immobilised antibodies in separation in an immunoassay may suffer from several disadvantages; for example interaction between antibody and analyte species (or authentic analyte species) may be considerably slower (e.g. due to diffusion considerations) than that which takes place with the same antibody and analyte species (or authentic analyte species) in solution (i.e. a longer time may be required to reach equilibrium).

Also, the immobilisation of antibody on a solid phase is not a problem-free procedure; thus, for example, it can be difficult to control or reproduce and also serious protein leakage may occur.

The present invention may offer the advantages of reactions in solution with the convenience of a rapid solid-phase separation technique.

Reference has hereinbefore been made to an entity to be detected; such an entity to be detected may be, for example, an analyte species-containing species (i.e. a species containing or carrying an analyte species) formed by interaction of an analyte species with a suitable agent.

Thus, in an embodiment of the present invention there is provided a method which includes the use of an agent, said agent being capable of interacting with an analyte species, or with an authentic analyte species, to form an entity to be detected, the use of a binding species for association with the agent, the use of an auxiliary species, said binding species and said auxiliary species being such as to be capable of binding together, the use of a support material, and the use of an antibody to the entity to be detected.

It is to be understood that the analyte species, or authentic analyte species, the agent, the binding species, the auxiliary species, the support material and the antibody may be brought together in any suitable manner and at any suitable time.

Thus, by way of example, the present invention provides a method which includes the steps of bringing together an analyte species and a binding species, carrying an agent capable of interaction with the analyte species, thereby to effect interaction of the analyte species with the agent so as to form an entity to be detected, bringing together the binding species, carrying the entity to be detected thus formed, and an auxiliary species provided on a support material, so as to cause binding between the binding species and the auxiliary species, and applying an antibody, said antibody being an antibody to the entity to be detected, such that the antibody binds with the entity to be detected.

By way of further example, as an alternative to the example immediately hereinbefore disclosed, the auxiliary species and the binding species, carrying an agent capable of interaction with an analyte species, may be arranged to bind together before an analyte species interacts with the agent to form the entity to be detected.

It will be appreciated that the antibody to the entity to be detected may be an antibody that is a specific antibody to the entity to be detected.

In accordance with the present invention an antibody to the entity to be detected may, optionally, carry a detectable species (as hereinbefore disclosed). Thus, for example, the detectable species may be a tracer species (e.g. an enzyme tracer, a fluorescence tracer or a radioactive tracer).

Accordingly, in accordance with an embodiment of the present invention there is provided a method which comprises the steps of bringing together a binding species, and an auxiliary species provided on a support material, so as to cause binding between the binding species and the auxiliary species, arranging for the binding species to carry an entity to be detected, applying an antibody, said antibody being an antibody to the entity to be detected and said antibody carrying a tracer species, such that the antibody binds with the entity to be detected and detecting by means of the tracer species the binding of the antibody and the entity.

The binding species may be arranged to carry an entity to be detected in any suitable manner and at any suitable time (e.g. before or after binding between the binding species and the auxiliary species).

Any suitable agent capable of forming an entity to be detected may be carried by the binding species in accordance with the present invention. An agent capable of forming a complex with the analyte species is an example of an agent which may find application in the formation of an entity to be detected in accordance with the present invention.

Where the analyte species is a metal (e.g. in the form of a metal ion) the agent carried by the binding species may be, for example, an agent capable of forming a metal-containing species (e.g. a complex of the metal).

Examples of agents for forming a complex with a metal are chelating agents.

Thus in one embodiment of the present invention a method is provided which comprises bringing together a binding species, carrying one, or more, molecules of a chelating agent, and an analyte species comprising metal ions to form a binding species carrying a metal-chelate complex as an entity to be detected, bringing together the binding species, carrying the metal-chelate complex, and an auxiliary species provided on a support material, so as to cause binding between the binding species and the auxiliary species, applying an antibody, said antibody being an antibody to the entity to be detected and said antibody carrying a detectable species, such that the antibody binds with the metal-chelate complex and detecting the binding of the antibody and the metal-chelate complex.

By way of example, the antibody may carry a detectable species comprising an enzyme tracer and detecting the binding of the antibody and the metal-chelate complex may be by means of an enzyme induced change.

The enzyme induced change may be, for example, a colour change, or a fluorescence change, or a luminescence change or an electro-chemical change.

It will be appreciated that the antibody may be such that it recognizes the metal-chelate complex but not chelating agent molecules which are not complexed with a metal ion.

Thus, the antibody may be specific with respect to the metal-chelate complex. Also, the antibody may be such as to be specific to a complex of a particular metal ion with a chelating agent such that it does not bind significantly with complexes comprising other metal ions complexed with the same chelating agent.

Examples of chelating agents for forming a metal chelate are:

ethylene diamine tetra acetate (EDTA), diethylene triamine penta acetate (DTPA), cyclohexylenedinitrilo tetra acetic acid (CDTA), 1-benzyl-EDTA, derivatives of 1-benzyl-EDTA, 8-hydroxyquinoline, derivatives of 8-hydroxyquinoline, and deferoxamine.

The agent may be carried by the binding species in any suitable manner (e.g. by covalent linkage between the agent and the binding species); by way of example one unit of an agent may be linked to the binding species or more than one unit of an agent may be linked to the binding species. Thus, for example, one molecule of an agent may be linked to a binding species or, for example, more than one molecule of an agent may be linked to a binding species.

From the foregoing disclosure it will be appreciated that in an embodiment of the present invention a method may include the step of forming a complex of an analyte species said complex thus formed providing an entity to be detected.

Also, from the foregoing disclosure it will be appreciated that in an embodiment of the present invention a method may include the step of linking, either directly, or indirectly, an agent to a binding species.

The present invention may find application in, for example, detection of metals (e.g. in the form of free, hydrated metal ions) in environmental, or clinical, or industrial samples.

It will be understood that the present invention may find application in, for example, the detection and determination of metals which can be considered to be toxic in the environment. Accordingly, the present invention may find application in environmental monitoring.

Thus, for example, a level of concentration of a metal ion (e.g. the concentration of a heavy metal ion), which may typically be in the ppb range, may be detected and determined in any suitable sample. Thus, for example, samples of water, soil, living products (such as plants (e.g. vegetables) or animals) or air may provide an analyte species, or an entity to be detected, for detection in accordance with the present invention.

Preferably sample collection procedures and any concentration procedures should be such as to minimise contamination from external sources.

By way of example, samples to be subjected to detection in accordance with the invention may be, conveniently, aqueous samples.

The following are examples of metals which may be detected:

calcium ($Ca^{II}$), iron ($Fe^{II}$, $Fe^{III}$), cobalt ($Co^{II}$, $Co^{III}$), aluminium ($Al^{III}$), zinc ($Zn^{II}$), lead ($Pb^{II}$), copper ($Cu^{II}$, $Cu^{I}$), cadmium ($Cd^{II}$), vanadium ($V^{II}$, $V^{III}$, silver ($Ag^{I}$, $Ag^{II}$), mercury ($Hg^{I}$, $Hg^{II}$), indium ($In^{III}$), manganese ($Mn^{II}$) and nickel (Ni$^{II}$).

It will be appreciated that an entity to be detected and an antibody to the entity may be brought together in any suitable manner; thus, for example, the bringing together of an entity to be detected and an antibody in accordance with the present invention may be effected in an aqueous medium.

As hereinbefore disclosed an antibody to an entity to be detected may be prepared by any suitable method, for example those known for the raising of polyclonal or monoclonal antibodies; thus, antibodies may be raised, for example, by immunising animals.

For example, an analyte species may be reacted with an agent so as to form an entity (e.g. a complex of the analyte species) and the entity may be conjugated to a carrier protein prior to being used to prepare an antibody to the entity.

It is to be understood that when being used to prepare an antibody, the entity may be regarded as a hapten.

By way of further example, an agent may be conjugated to a carrier protein and then an analyte species introduced so as to react with the agent to form an entity (e.g. a complex of the analyte species) such that the carrier protein carrying the entity may be used to prepare an antibody to the entity.

Thus, for example, a chelating agent such as p-amino benzyl-EDTA may be conjugated to bovine serum albumin (BSA) or keyhole limpet haemocymin (KLH) via a thiocyanate-derivative or diazo-derivative of the p-amino function and the product resulting from this conjugation allowed to react with excess of a salt of a metal (e.g. lead nitrate or cobalt nitrate) so that a metal ion-EDTA-BSA species forms or a metal ion-EDTA-KLH species forms.

Following removal of excess metal salt (e.g. by gel filtration or dialysis) such a species may be used to raise an antibody to the metal ion-EDTA complex, for example, by immunising animals over a period of 4-6 months.

It will be appreciated that after the binding of a binding species with an auxiliary species provided on the support material, the binding species is also, in effect, attached to the support material. Thus, where, for example, a binding species is attached to an analyte species, or an entity to be detected, and an antibody to the analyte species, or to the entity to detected, is applied, bound antibody may be, in effect, attached to the support material (via, in turn, the analyte species or the entity to be detected, the binding species and the auxiliary species).

Thus, any required washing steps (e.g. with appropriate buffers) may be used to remove unbound materials.

Tracer species left on the support material, the amount of which is directly proportional to the concentration of the analyte species, or the entity to be detected, may be measured by any suitable method such as those known in the art.

It is to be understood that by use of an antibody to an entity to be detected (e.g. a metal complex), the binding of antibody and entity may be specific such that the detection thus achieved is specific and sensitive to the entity and thus to any analyte species which is part of the entity.

By way of example, analyte species concentrations in a nanomolar to picomolar (i.e. $10^{-9}$ to $10^{-12}$ M) range may be detected in accordance with the present invention (e.g. using an immunoassay procedure such as an enzyme-labelled immunoassay procedure).

Sensitivity of detection in accordance with the present invention may be expressed as a "detection limit" (also known in the art as a "quantification limit"). This limit relates to the relation between a change in a response and a concentration of an analyte species causing the change in response.

The "detection limit" is the lowest concentration or amount of an analyte species (usually expressed in concentration units) which can be detected by an analytical method.

The "detection limit" should, in general, have at least 95% confidence to be meaningful.

In one example of a method for detection in accordance with the present invention, which example includes the use of a labelled immunoassay procedure, factors which affect sensitivity of detection include:

(a) affinity of an antibody with respect to an analyte species or an entity to be detected,
(b) specific activity of any assay tracer used,
(c) signal species nature (i.e. the type of signal species used (e.g. radioactive tracer, enzymatic tracer or fluorescence tracer)),
(d) assay background (i.e. background "noise") and (e) instrumentation used to measure assay signal.

Where, for example, an entity to be detected is to be detected in accordance with the present invention, it will be appreciated that the entity to be detected should be sufficiently stable to permit satisfactory detection to be effected. It will also be appreciated that the entity may be considered to be acting as a "hapten" with respect to the antibody to the entity.

Where in accordance with the present invention an entity to be detected and an antibody to the entity are utilised, specificity in relation to detection of analyte species, which forms part of the entity to be detected, in accordance with the present invention may be defined as the degree to which the detection distinguishes between the analyte species (e.g. metal ion) and other species (e.g. other metal ions) when a sample to be subjected to detection contains a mixture of species.

The specificity of detection may, therefore, depend upon the specificity of the antibody to the entity to be detected in the presence of other species (e.g. a complex or complexes which may give rise to interfering reactions).

For example, an agent capable of forming a complex with an analyte species may also be capable of forming a complex with species other than the analyte species.

In such circumstances there may be some "cross-reaction" of antibody (i.e. antibody to the complex of the analyte species) with complexes of an agent with species other than the analyte species.

By way of example, one procedure for determining cross-reaction of an antibody makes use of an immobilised primary antibody and labelled complex of the analyte species in a competitive immunoassay method. Thus, a calibration curve (i.e. a standard curve) may be constructed using a range of analyte species concentrations and a "limited" amount of immobilised antibody. Binding of a limited amount of labelled complex of the analyte species is monitored in the absence of a competing standard complex of the analyte species and in the presence of increasing levels of such a standard.

A calibration curve (a "dose response curve") is constructed by plotting the level of bound tracer (as % bound) against the concentration of standard. It is to be understood that the binding of the tracer may be taken as 100% (when 0% of the standard is bound) or the total amount of tracer may be taken as 100%.

The above steps are repeated replacing the standard with a range of known concentrations of complexes comprising the same agent (as used in complexing the analyte species) complexed with species other than the analyte species; all other parameters are kept substantially unchanged.

Thus, a series of dose response curves of standard and possible "cross-reacting" complexes may be co-plotted on a graph.

Cross-reaction values (CR) are calculated as 100 x the ratio of molar concentrations of standard and possible cross-reacting complexes which cause a decrease of tracer binding by 50% (taking the binding value in the absence of competing entities as 100%).

Accordingly,

$$CR = \frac{[\textit{moles of standard complex}]_{50\%}}{[\textit{moles of cross-reacting complex}]_{50\%}} x\ 100.$$

From the foregoing disclosure it will be understood that, by way of example, detection of an analyte species in accordance with the present invention may be conducted using an immunochemical detection procedure, for example, an immunoassay procedure.

Where, for example, an agent capable of interacting with an analyte species is used to form an entity to be detected, an analyte species (e.g. a metal ion) may be, for example, extracted from a sample by interaction with the agent which may be associated with a binding species thereby to form an entity to be detected.

Subsequently, the binding species may be bound to an auxiliary species provided on a support material and then a labelled antibody is applied; the amount of entity present (and hence the amount of analyte species present in the sample) may be determined by methods known for immunoassay.

The present invention may also offer for sensors used in accordance with the present invention, for example immunosensors such as optical immunosensors and electrochemical immunosensors, the possibility that a sensor surface carrying an auxiliary species may be the same for a variety of, or all, analyte species thereby providing a generic sensor construction.

A combination comprising a sensor surface carrying an auxiliary species may also, for example, be such as to permit regeneration for multiple reuse; for example, such a combination of a sensor surface carrying an auxiliary species may be regenerated by removal of bound substances by washing with a suitable buffer preparation (e.g. 0.1M glycine-HCl at pH 2.5).

Also, by way of further example, the auxiliary species may be selected so as to be stable to multiple treatment with low or high pH organic solvents and/or chaiotropic reagents thereby to permit regeneration for multiple reuse.

The present invention thus offers the possibility of constructing a non-dedicated sensor such as an immunosensor or immunoelectrode.

Thus, by way of example, an electrochemical immunosensor may be provided in which an electrode surface is coated with a conducting polymer (e.g. a polypyrrole polymer) which may be arranged to act as an auxiliary species. For use in such a sensor antibodies to the conducting polymer (e.g. polypyrrole polymer), or segments thereof, may be generated by suitable techniques such as those known in the art; a combination comprising an electrode surface and a conducting polymer coating may be, for example, such as to permit regeneration for multiple reuse.

The methods of competitive and non-competitive immunoassay, hereinbefore described by way of example, may be used in an electrochemical immunosensor as immediately hereinbefore described. A tracer species for use in such an electrochemical immunosensor may be arranged to provide an electroactive species.

In conventional electrochemical immunosensors known in the art specific antibodies may be immobilised on an electrode surface thereby to provide a dedicated immunosensor; such immunosensors may have inherent constraints when multiple electrodes with the same properties are required. Thus, disadvantages may become apparent since regeneration of the sensor may be difficult or regeneration may lead to deterioration of antibody activity.

The present invention may offer the possibility of avoiding such disadvantages in that a sensor used in accordance with the present invention may not need to be a dedicated sensor.

It will be appreciated from the foregoing that, by way of further example, detection of an analyte species in accordance with the present invention may use an immunosensor technique.

For example, the auxiliary species may be immobilised on a surface of a sensor device (e.g. on a glass surface, on a quartz surface, or on the surface of an electrode).

For example, in the use of direct optical immunosensors no label is used and the binding of an analyte species or entity to be detected and antibody may give rise to signal generation to permit detection (e.g. by surface plasmon resonance).

By way of further example, in the use of a labelled antibody immunosensor, signal generation may be detected by a suitable method appropriate to the nature of the signal generated by the label (e.g. a means for measuring fluorescent emission may be used when the label is a fluorescence producing entity).

According to a further aspect of the present invention, there is provided use of a test-kit, which test-kit includes a support material and an auxiliary species provided on said support material, in a method in accordance with the present invention.

In one embodiment the binding species may carry an agent capable of forming an entity to be detected when contacted with an analyte species and an antibody to the entity may be used; a means for determining by an immunological procedure, the amount of entity formed and hence the amount of analyte species present in a given sample (e.g. an aqueous sample) may be also used.

It will be appreciated that detection in accordance with the present invention may be, for example, qualitative or quantitative; it will also be appreciated that quantitative detection may be used to determine a level of concentration.

Also, it will be appreciated that the present invention may be utilised with samples which contain analyte species or entity to be detected and with samples which contain substantially no analyte species or no entity to be detected (e.g. a "blank" standard sample or an "unknown" sample which, upon subjecting to detection in accordance with the present invention, is found to contain substantially no analyte species or entity to be detected).

Further it will be appreciated, by way of example, that by use of standard quantities of authentic analyte species, or authentic entity to be detected, calibration may be effected such that, subsequently, unknown quantities of analyte species, or unknown quantities of entity to be detected may be determined.

The present invention will now be further described, by way of example only, as follows:

Example 1

**Preparation of anti-17$\beta$-estradiol-antiserum**

17$\beta$-estradiol was selected as an analyte species to illustrate, by way of example, the use of an auxiliary species-binding species pair system in the separation, in accordance with the present invention, of bound and unbound fractions in ELISA (enzyme labelled immunosorbent assay) and, accordingly, it was necessary to prepare an antibody to 17$\beta$-estradiol.

17$\beta$-estradiol-3-(O-carboxymethyl ether), (i.e. $E_2$3CME), was prepared and coupled to keyhole limpet haemocyanin (KLH) to synthesise an immunogen. Thus, 17$\beta$-estradiol-3-(O-carboxymethyl ether), was derivatised to its N-hydroxysuccinimide ester. The ester (10 mg/ml in dioxane), was added to 50 mg of KLH (in 10 ml of 0.1M NaHCO$_3$, pH 8.6). The resulting conjugate immunogen was purified by dialysis against 3% NaHCO$_3$. Rabbit antiserum to 17$\beta$-estradiol was generated by the injection of 0.5 mg quantities of the conjugate immunogen at monthly intervals for 9 months. Estradiol derivative, as hereinbefore described, was also coupled to egg albumin (ovalbumin) to prepare an authentic analyte species-protein conjugate capable of being adsorbed onto polystyrene microtitre plates.

Crystalline egg albumin, 30 mg in 6 ml of 0.1M NaHCO$_3$ (pH 8.6), was reacted with 0.3 mg of $E_2$3CME active ester (in 1 ml of dimethylformamide). The conjugate thus formed was purified by dialysing extensively against 3% NaHCO$_3$ and then treated with activated charcoal (1%) to remove uncoupled estradiol.

**Titration of binding activity of anti-17$\beta$-estradiol antibody**

96-well polystyrene microtitre ELISA plates were coated with the authentic analyte-protein conjugate (i.e.

E$_2$3CME-ovalbumin) prepared as hereinbefore disclosed. Thus, the conjugate (0.1 μg in 100 μl in 0.1M NaHCO$_3$) was allowed to adsorb for 24 hrs. Excess sites were blocked by a 0.5 g.l$^{-1}$ solution of horse haemoglobin (150 μl in 0.1M NaHCO$_3$). Antibody binding activity in the anti-17β-estradiol antiserum was assessed by antibody dilution response graphs. The antiserum was serially diluted in assay buffer (50mM Tris-HCl, pH 7.4, containing 0.1M NaCl, 0.1% gelatin, 0.05% Tween (Registered Trade Mark) 20 and 0.001% thimerosal) and 100 μl volumes of each dilution thus obtained were added (in duplicates) to the coated wells of the coated plates prepared as hereinbefore described and allowed to react at 37°C for 2 hrs. After washing the plates, 100 μl of second antibody-horse radish peroxidase conjugate was added and left to react for a further 1 hr at 37°C. (The second antibody - horse radish peroxidase conjugate was a commercially available conjugate comprising a con-jugate of donkey anti-rabbit immunoglobulin antibody and horse radish peroxidase.) Assay signal was developed by the horse radish peroxidase (HRP) substrate 2,2'-azino-bis(3-ethylbenzo-thiazoline-6-sulphonic acid) (ABTS) using 20 min reaction time. The optical density (O.D.) at 415 nm was read in a standard ELISA plate reader.

The results are shown in Table I.

Table I

| Antiserum dilution | O.D. (average of 2 readings) |
|---|---|
| 1/500 | 2.90 |
| 1/4500 | 2.60 |
| 1/13500 | 2.00 |
| 1/40500 | 1.35 |
| 1/121500 | 0.64 |
| 1/364500 | 0.300 |

Example 2

**Preparation of antiserum to auxiliary species**

In this Example antibody (a binding species) to an auxiliary species, being a ligand, was prepared. Thus, 7-amino 4-methyl coumarin-3-propionic acid (7AMCPA) was synthesised and coupled to bovine serum using active ester derivative to form an immunogen conjugate. Sheep antibody to 7-amino 4-methyl coumarin was generated by the injection of 1.5 mg quantities of the immunogen conjugate at monthly intervals for 12 months. 7-amino 4-methyl coumarin ester derivative, as hereinbefore disclosed, was coupled to egg albumin (ovalbumin) to provide a ligand-protein conjugate for use in the coating of polystyrene microtitre plates. Antibody binding activity in the generated antiserum was assessed by using a dilution response curve procedure involving ELISA as disclosed in Example 1 with the exception that 2 μg of ligand-protein conjugate were used to coat the plates and anti-sheep antibody-HRP conjugate was used as the second antibody. The results are shown in Table II.

It will be appreciated that the antibody to the auxiliary species is the binding species for the auxiliary species.

Table II

| Antiserum dilution | O.D. (average of two readings) |
|---|---|
| 1/500 | > 3.0 |
| 1/4500 | > 3.0 |
| 1/13500 | > 3.0 |
| 1/40500 | 2.80 |
| 1/121500 | 1.69 |
| 1/364500 | 0.92 |
| 1/1.09 x 10$^6$ | 0.31 |

Example 3

**coupling of analyte derivative to the binding species**

The IgG fraction of sheep anti-7AMCPA antibody was prepared by ion exchange chromatography on DEAE-Sephadex A50. (Sephadex is a Registered Trade Mark.) 17β-estradiol-3-(O-carboxymethyl ether) was coupled to the IgG fraction by standard conjugation methods. Thus, 2.0 mg of $E_2$3CME-active ester (in 200 µl of dioxane) were added to 100 mg of the IgG fraction in 15 ml of 0.1M NaHCO$_3$ (pH 8.6) to form a binding species-authentic analyte species conjugate. After dialysis and treatment with 1% charcoal the antibody binding activity in this conjugate was assessed by an ELISA procedure similar to that disclosed in Example 2.

The results are shown in Table III.

Table III

| Conjugate dilution | O.D. (average of 2 readings) |
|---|---|
| 1/1000 | > 3.0 |
| 1/3000 | > 3.0 |
| 1/9000 | 2.7 |
| 1/27000 | 1.8 |
| 1/81000 | 1.1 |
| 1/243000 | 0.5 |

Example 4

**Purification of binding species-authentic analyte species conjugate**

Binding species-authentic analyte species conjugate, prepared by coupling $E_2$3CME-active ester to the IgG fraction of sheep anti-7AMCPA antibody, as disclosed in Example 3, contained non-antibody IgG conjugates as contaminants. These contaminants were removed by affinity chromatography purification using the auxiliary species disclosed in Example 2 coupled to Sepharose-6B as the affinity matrix. Thus an affinity matrix was prepared by coupling a 7AMCPA-egg albumin (ovalbumin) conjugate (20 mg) to cyanogen bromide-activated Sepharose-6B (5 ml). (Sepharose is a Registered Trade Mark.)

All steps in the treatment of the affinity matrix, its washing, and the affinity chromatography procedure were carried out in the cold (5°C) and a cold chromatography cabinet was used throughout.

The affinity matrix was first washed extensively according to standard procedure in affinity chromatography. In order to remove loosely bound ligand from the matrix, the matrix was mixed with 5 ml of anti-7AMCPA antiserum (diluted with assay buffer to 20 ml), and subsequently the matrix was washed with 100 ml of eluting reagent (20% acetonitrile and 1% propionic acid in distilled water). The affinity matrix was washed thoroughly with assay buffer to remove traces of the eluting reagent; the assay buffer had the composition disclosed in Example 1.

It will be appreciated that the affinity matrix thus prepared may be referred to as 7AMCPA-ovalbumin-Sepharose-GB gel. (Sepharose is a Registered Trade Mark.)

Anti-7AMCPA antibody-$E_2$3CME conjugate (80 mg in 20 ml of buffer, 0.1M phosphate buffer, pH 7.1, containing 0.1M NaCl, 0.1% sodium azide and 0.05% Tween (Registered Trade Mark) 20) was passed through 2 ml of the affinity matrix in a column at a flow rate of about 0.1 ml/min. After all of the conjugate had been exposed to the matrix in the column, by being introduced in solution to the column, the column was washed with 50 ml of wash buffer (0.1M phosphate buffer, pH 7.5, containing 0.5M NaCl, 0.05% Tween 20 and 0.1% sodium azide) to remove unbound materials. When the optical density (at 280 nm) of eluate from the column fell to 0.05, the column was washed with 20 ml of ice-cold distilled water, the flow rate of which was increased to 0.5 ml/min during washing steps. After the water wash, a gradient of the eluting reagent was applied to the column. (The eluting reagent was a mixture of 20% acetonitrile and 1% propionic acid in distilled water.) The eluting reagent was kept in ice to maintain low temperature. Eluted fractions (2 ml per fraction) were received in 0.5 ml of 0.2M phosphate buffer (pH 8.0) to neutralise the acid. The elution was followed by measuring O.D. at 280 nm (protein absorption). The results are shown in Table IV.

Table IV

| Fraction No. | O.D. | Fraction No. | O.D. |
|---|---|---|---|
| 1 | 0.04 | 14 | 0.05 |
| 2 | 0.05 | 15 | 0.05 |
| 3 | 0.05 | 16 | 0.15 |
| 4 | 0.05 | 17 | 0.78 |
| 5 | 0.06 | 18 | 1.78 |
| 6 | 0.06 | 19 | 1.90 |
| 7 | 0.06 | 20 | 1.90 |

Table IV   (continued)

| Fraction No. | O.D. | Fraction No. | O.D. |
|---|---|---|---|
| 8 | 0.07 | 21 | 1.0 |
| 9 | 0.05 | 22 | 0.8 |
| 10 | 0.05 | 23 | 0.3 |
| 11 | 0.04 | 24 | 0.6 |
| 12 | 0.05 | 25 | 0.15 |
| 13 | 0.04 | 26 | 0.08 |

(The O.D. figures for Fractions 1 to 15 represent those of essentially distilled water since eluting reagent was first applied to the column at approximately the time Fraction 11 was being collected from the column. It will be noted that an increasing content of eluting agent gives rise to elution of protein.)

The Fractions containing protein (Fractions 17 to 24) were pooled and dialysed extensively against 0.1M sodium phosphate buffer (pH 7.4) + 0.1M NaCl in the cold (5°C) to reduce the concentration of eluting reagent and to allow the antibody to renature.

Example 5

The antibody binding activity in the post-dialysis affinity purified conjugate (as prepared in accordance with Example 4) was assessed by a dilution response curve procedure similar to that disclosed in Example 2. The results are shown in Table V.

Table V

| Dilution of anti-7AMCPA antibody-$E_2$3CME conjugate | O.D. (with anti-sheep antibody (HRP)) |
|---|---|
| 1/10 | > 3.0 |
| 1/100 | > 3.0 |
| 1/300 | > 3.0 |
| 1/900 | 2.9 |
| 1/2700 | 2.9 |
| 1/8100 | 2.6 |
| 1/24300 | 1.5 |
| 1/72900 | 0.74 |

Example 6

The estradiol content of the conjugate as prepared in accordance with Example 4 was titrated by first allowing the conjugate to bind to ligand adsorbed on microtitre plates, washing with buffer, applying rabbit anti-estradiol antibody (diluted to 1/10,000) and allowing to react for 1 hr at 37°C. After washing, a signal was developed by anti-rabbit antibody-HRP conjugate using a procedure similar to that disclosed in Example 1. The results are shown in Table VI.

Table VI

| Dilution of anti-7AMCPA antibody-$E_2$3CME conjugate | O.D. (with anti-$E_2$3CME antibody + anti-rabbit antibody-HRP |
|---|---|
| 1/10 | > 0.3 |
| 1/100 | > 0.3 |
| 1/300 | > 0.3 |
| 1/900 | 2.7 |
| 1/2700 | 2.3 |
| 1/8100 | 1.7 |
| 1/24300 | 1.0 |
| 1/72900 | 0.5 |

Example 7

A calibration graph for the analyte species estradiol was constructed by ELISA using a separation in accordance with the present invention. Excess auxiliary ligand (7AMCPA-egg albumin) was adsorbed to ELISA microtitre plates as disclosed in Example 2.

Estradiol standards were prepared in assay buffer; the assay buffer was of the composition given in Example 1. Duplicate 50 µl volumes of this standard were placed in glass test tubes, to which were added the following: 50 µl of affinity chromatography purified anti-7AMCPA antibody-E$_2$3CME conjugate, prepared as disclosed in Example 4, (diluted to 1/7000) and 50 µl of rabbit anti-E$_2$3CME antibody (diluted to 1/10,000). After incubation for 30 mins at 37°C, a 100 µl volume from the contents of each assay tube was transferred to individual wells of a microtitre plate carrying auxiliary species which was, in this Example, the ligand 7-amino-4-methyl-coumarin-3-propionic acid. In this Example, the ligand was conjugated with ovalbumin and was thus indirectly linked to the support material (microtitre plate) via ovalbumin.

After incubation at 37°C for 30 min, the microtitre plate was washed and anti-rabbit antibody-HRP conjugate was added. The assay was completed as disclosed in Example 1.

The results are shown in Table VII.

Table VII

| Concentration Analyte Species (Estradiol) (ng/tube) | O.D. at 415 nm (average of 2) |
|---|---|
| 0 | 1.75 |
| 0.039 | 1.25 |
| 0.078 | 0.98 |
| 0.156 | 0.80 |
| 0.312 | 0.68 |
| 0.625 | 0.51 |
| 1.25 | 0.39 |
| 2.50 | 0.29 |
| 5.00 | 0.20 |
| 10.00 | 0.16 |
| Blank (primary antibody) | 0.10 |

Example 8

A cross-reaction graph for estrone (a major cross-reactant with respect of estradiol) was constructed by a procedure as disclosed in Example 7 using estrone in place of estradiol.

The results are given in Table VIII.

Table VIII

| Concentration Estrone (ng/tube) | O.D, at 415 nm (average of 2) |
|---|---|
| 0 | 1.76 |
| 0.039 | 1.47 |
| 0.078 | 1.36 |
| 0.156 | 1.25 |
| 0.312 | 1.14 |
| 0.625 | 1.10 |
| 1.25 | 0.95 |
| 2.50 | 0.86 |
| 5.0 | 0.68 |
| 10.00 | 0.55 |
| Blank (primary antibody) | 0.09 |

Example 9

A cross-reaction graph for estriol (a major cross-reactant with respect to estradiol) was constructed by a procedure as disclosed in Example 7 using estriol in place of estradiol.

Table IX

| Concentration Estriol (ng/tube) | O.D. at 415 nm (average of 2) |
|---|---|
| 0 | 1.76 |
| 0.039 | 1.49 |
| 0.078 | 1.44 |
| 0.156 | 1.28 |
| 0.312 | 1.20 |
| 0.625 | 1.16 |
| 1.25 | 1.00 |
| 2.50 | 0.90 |
| 5.00 | 0.80 |
| 10.00 | 0.67 |
| Blank (primary antibody) | 0.10 |

On comparing the results obtained in Examples 7, 8 and 9 it will be seen that the response for the analyte species (estradiol) differs significantly from the two major cross-reactants estrone and estriol.

**Claims**

1. A separation method, suitable for use in an immunoassay method for the detection of an analyte species, which separation method is **characterised in that** said separation method includes arranging for an auxiliary species and a binding species for the auxiliary species to undergo specific binding, said auxiliary species not being a primary species and said auxiliary species being a ligand and said auxiliary species being provided on a support material, said support material being substantially non-permeable, said binding species being an antibody capable of specific binding with the auxiliary species and said binding species being capable of being linked with a primary species by means of a linkage which involves a link, of a non-specific binding type, to the binding species, said binding species being arranged to be linked with a primary species by means of said linkage.

2. A method as claimed in Claim 1 further **characterised in that** the link of a non-specific binding type is a covalent link or a link which involves adsorption.

3. A method as claimed in Claim 1 or claim 2 further **characterised in that** the linkage also includes a further link of a non-specific binding type or a further link of a specific binding type.

4. A method, suitable for use in the detection of an analyte species by immunoassay, which method is **characterised in that** said method includes arranging for an auxiliary species and a binding species for the auxiliary species to undergo specific binding, said auxiliary species not being a primary species and said auxiliary species being a ligand and said auxiliary species being provided on a support material, said support material being substantially non-permeable, said binding species being an antibody capable of specific binding with the auxiliary species and said binding species being capable of being linked with a primary species by means of a linkage which involves a link, of a non-specific binding type, to the binding species, said binding species being arranged to be linked with a primary species by means of said linkage.

5. A method as claimed in any one of Claims 1 to 3 further **characterised in that** the method includes the use of an auxiliary species, being a ligand, provided on a support material, the use of a binding species for the auxiliary species, said binding species being an antibody capable of specific binding with the auxiliary species and said binding species being capable of being linked with a primary species by means of a linkage which involves a link, of a non-specific binding type, to the binding species, and the use of an antibody to an analyte species or the use of an antibody to an entity to be detected.

6. A method as claimed in Claim 5 further **characterised in that** the method includes the use of an auxiliary species, being a ligand, provided on a support material, the use of a binding species for the auxiliary species, said binding species being an antibody capable of specific binding with the auxiliary species and said binding species carrying an entity to be detected, and the use of an antibody to the entity to be detected.

7. A method as claimed in Claim 4 further **characterised in that** the method includes the use of an auxiliary species, being a ligand, provided on a support material, the use of a binding species for the auxiliary species, said binding species being an antibody capable of specific binding with the auxiliary species and said binding species being capable of being linked with a primary species by means of a linkage which involves a link, of a non-specific binding type, to the binding species, and the use of an antibody to an analyte species or the use of an antibody to an entity to be detected.

8. A method as claimed in Claim 7 further **characterised in that** the method includes the use of an auxiliary species, being a ligand, provided on a support material, the use of a binding species for the auxiliary species, said binding species being an antibody capable of specific binding with the auxiliary species and said binding species carrying an entity to be detected, and the use of an antibody to the entity to be detected.

9. A method as claimed in any one of the preceding claims further **characterised in that** the support material, or a part thereof, provides the auxiliary species.

10. A method as claimed in any one of Claims 1 to 8 further **characterised in that** the auxiliary species is attached directly, or indirectly, to the support material.

11. A method as claimed in Claim 9 or Claim 10 further **characterised in that** an oligomer of an auxiliary species or a polymer of an auxiliary species is provided on the support material.

12. A method as claimed in any one of the preceding Claims further **characterised in that** the auxiliary species is 2,4 dinitrophenol, fluorescein, digitoxin, coumarin or cibacron blue.

13. A method as claimed in any one of claims 1 to 11 further **characterised in that** the auxiliary species is a polypeptide, a protein, a polysaccharide or a conducting polymer.

14. A method as claimed in any one of the preceding Claims further **characterised in that** the auxiliary species is provided as a coating of a polymer on a support material.

15. A method as claimed in any one of the Claims 1 to 12 or Claim 14 further **characterised in that** the binding species is anti-fluorescein antibody, anti-coumarin antibody, anti-2,4 dinitrophenol antibody or anti-cibacron blue antibody.

16. A method as claimed in any one of the preceding Claims further **characterised in that** the primary species is a primary antibody or a ligand.

17. A method as claimed in any one of the preceding Claims further **characterised in that** the analyte species is detectable as such or wherein the analyte species is part of an entity to be detected.

18. A method as claimed in Claim 17 further **characterised in that** the analyte species is a hormone, a drug, a steroid, a tumour marker, human serum albumin, human chlorionic gonadotrophin, human IgG, a protein antigen, a blood protein, a marker protein, a toxin, a micro-organism, a metal complex or a metal ion.

19. A method as claimed in Claim 18 further **characterised in that** the metal complex is methyl mercury.

20. A method as claimed in claim 18 further **characterised in that** the metal ion is a calcium ion, an iron ion, a cobalt ion, an aluminium ion, a zinc ion, a lead ion, a copper ion, a cadmium ion, a vanadium ion, a silver ion, a mercury ion, an indium ion, a manganese ion or a nickel ion.

21. A method as claimed in any one of the preceding Claims further **characterised in that** an entity to be detected is formed by interaction of an analyte species with an agent.

22. A method as claimed in Claim 21 further **characterised in that** the agent is an agent capable of forming a complex.

23. A method as claimed in Claim 21 or Claim 22 further **characterised by** including the step of forming a complex of an analyte species said complex providing an entity to be detected.

24. A method as claimed in any one of claims 21 to 23 further **characterised by** including the use of an agent, said agent being capable of interacting with an analyte species, or with an authentic analyte species, to form an entity to be detected, the use of a binding species for association with the agent, the use of an auxiliary species, said binding species being a binding species for the auxiliary species and said binding species and said auxiliary species being such as to be capable of binding together, the use of a support material, and the use of an antibody to the entity to be detected.

25. A method as claimed in any one of Claims 21 to 24 which method is further **characterised by** including the steps of bringing together an analyte species and a binding species, carrying an agent capable of interaction with the analyte species, thereby to effect interaction of the analyte species with the agent so as to form an entity to be detected, bringing together the binding species, carrying the entity to be detected thus formed, and an auxiliary species provided on a support material, so as to cause binding between the binding species and the auxiliary species, and applying an antibody, said antibody being an antibody to the entity to be detected, such that the antibody binds with the entity to be detected.

26. A method as claimed in any one of Claims 21 to 24 further **characterised in that** the auxiliary species and a binding species, carrying an agent capable of interaction with an analyte species, are arranged to bind together before an analyte species interacts with the agent to form an entity to be detected.

27. A method as claimed in any one of Claims 21 to 26 further **characterised by** comprising the steps of bringing together a binding species and an auxiliary species provided on a support material, so as to cause binding between the binding species and the auxiliary species, arranging for the binding species to carry an entity to be detected, applying an antibody, said antibody being an antibody to the entity to be detected and said antibody carrying a tracer species, such that the antibody binds with the entity to be detected and detecting by means of the tracer species the binding of the antibody and the entity.

28. A method as claimed in any one of Claims 21 to 27 further **characterised in that** a chelating agent is used to form a complex as an entity to be detected.

29. A method as claimed in any one of Claims 21 to 28 further **characterised by** comprising bringing together a binding species, carrying one, or more, molecules of a chelating agent, and an analyte species comprising metal ions to form a binding species carrying a metal-chelate complex as an entity to be detected, bringing together the binding species, carrying the metal-chelate complex, and an auxiliary species provided on a support material, so as to cause binding between the binding species and the auxiliary species, applying an antibody, said antibody being an antibody to the entity to be detected and said antibody carrying a detectable species, such that the antibody binds with the metal-chelate complex and detecting the binding of the antibody and the metal-chelate complex.

30. A method as claimed in Claim 29 further **characterised in that** the antibody carries a detectable species comprising an enzyme tracer and the binding of the antibody and the metal-chelate complex is detected by an enzyme induced change.

31. A method as claimed in Claim 30 further **characterised in that** the enzyme induced change is a colour change, a fluorescence change, a luminescence change or an electro-chemical change.

32. A method as claimed in Claim 28 or Claim 29 further **characterised in that** the chelating agent is ethylene diamine tetra acetate, diethylene triamine penta acetate, cyclohexylenedintrilo tetra acetic acid, 1-benzyl-ETDA, a derivative of 1-benzyl-EDTA, 8-hydroxyquinoline, a derivative of 8-hydroxyquinoline, or deferoxamine.

33. A method as claimed in any one of the preceding Claims further **characterised in that** the support material is a solid phase material and the solid phase material is a reaction vessel wall, an insoluble polysaccharide, a microparticle, polystyrene, cross-linked dextran, an insoluble polymer structure, a glass surface, a derivatised silica surface, a polymer attached to a surface, a microparticulate material with entrapped ferrous oxide, nylon or a polyamide.

34. A method as claimed in any one of Claims 1 to 28, or 32, or 33 further **characterised in that** a detectable species is used.

35. A method as claimed in Claim 34 further **characterised in that** a detectable species is used and the detectable species comprises an enzyme, a fluorophore, a polymeric fluorophore, a radioisotope, a ligand, a polymer of a ligand, or a binder.

36. A method as claimed in Claim 35 further **characterised in that** a detectable species is a ligand and the ligand is detected by use of a binder therefor, or the detectable species is a binder and the binder is detected by use of a ligand therefor.

37. A method as claimed in any one of the preceding Claims further **characterised in that** a tracer species is used.

38. A method as claimed in Claim 37 further **characterised in that** the tracer species is an enzyme, a fluorophore, a chemiluminescent compound, a bioluminescent compound, a radioisotope or a dye.

39. A method as claimed in Claim 38 further **characterised in that** the tracer species is alkaline phosphatase, β-galactosidase, horse-radish peroxidase, a fluorescein, a coumarin, or rhodamin.

40. A method as claimed in any one of Claims 1 to 8 or 10 to 39 further **characterised by** including attaching an auxiliary species to a support material.

41. A method as claimed in any one of Claims 1 to 8, or 10 to 40 further **characterised by** including the step of linking, either directly, or indirectly, an agent to a binding species.

42. A method as claimed in any one of the preceding Claims further **characterised in that** a sample of water, soil, a living species, or air provides an analyte species, or an entity to be detected, for detection.

43. A method as claimed in any one of the preceding Claims further **characterised in that** a biological sample is subjected to detection.

44. A method as claimed in Claim 43 further **characterised in that** the biological sample is blood, plasma, serum, urine, saliva or milk.

45. A method as claimed in any one of the preceding Claims further **characterised in that** an analyte species, or an entity to be detected, is present in water, an aqueous preparation, or a fluid extract.

46. A competitive immunoassay method which method is **characterised by** including the use of a separation method as claimed in Claim 1.

47. A non-competitive immunoassay method which method is **characterised by** including the use of a separation method as claimed in Claim 1.

48. Use of a sensor, **characterised in that** said sensor includes a support material and an auxiliary species provided on said support material, in a method according to any one of Claims 4 to 47.

49. Use of a support material, **characterised in that** an auxiliary species is provided on said support material, in a separation method according to any one of Claims 1 to 3 or Claims 5 to 47.

50. Use of a support material, **characterised in that** an auxiliary species is provided on said support material, in a method according to any one of claims 4 to 47.

51. Use of a test-kit, **characterised in that** said test-kit includes a support material and an auxiliary species provided on said support material, in a method according to any one of Claims 4 to 47.

**Patentansprüche**

1. Trennverfahren, geeignet zur Verwendung in einem Immunoassay-Verfahren zur Detektion einer zu analysierenden Spezies, wobei das Trennverfahren **dadurch gekennzeichnet ist, daß** das Trennverfahren die Bereitstellung einer Hilfsspezies und einer Bindespezies für die Hilfsspezies beinhaltet, um eine spezifische Bindung einzugehen, wobei die Hilfsspezies keine Primärspezies ist und die Hilfsspezies ein Ligand ist und die Hilfsspezies auf einem Trägermaterial bereitgestellt wird, wobei das Trägermaterial im wesentlichen nicht durchlässig ist, es sich bei der Bindespezies um einen Antikörper handelt, der fähig ist, eine spezifische Bindung mit der Hilfsspezies einzugehen, und die Bindespezies geeignet ist, mit Hilfe einer Verknüpfung, die eine Bindung eines nicht spezifischen Bindungstyps an die Bindespezies beinhaltet, an eine Primärspezies gebunden zu werden, wobei die Bindespezies so angeordnet ist, daß sie mit Hilfe der Verknüpfung an die Primärspezies gebunden wird.

2. Verfahren nach Anspruch 1,
ferner **dadurch gekennzeichnet, daß** die Verknüpfung des nicht spezifischen Bindungstyps eine kovalente Bindung oder eine Adsorption beinhaltende Verknüpfung ist.

3. Verfahren nach Anspruch 1 oder 2,
ferner **dadurch gekennzeichnet, daß** die Verknüpfung auch eine weitere Bindung eines nicht spezifischen Bindungstyps oder eine weitere Bindung eines spezifischen Bindungstyps beinhaltet.

4. Verfahren, geeignet zur Verwendung bei der Detektion einer zu analysierenden Spezies mittels Immunoassay, wobei das Verfahren
**dadurch gekennzeichnet**
**ist, daß** das Verfahren beinhaltet, eine Hilfsspezies und eine Bindespezies für die Hilfsspezies bereitzustellen, so daß diese eine spezifische Bindung eingehen, wobei es sich bei der Hilfsspezies nicht um eine Primärspezies handelt und die Hilfsspezies ein Ligand ist und die Hilfsspezies auf einem Trägermaterial aufgebracht ist, wobei das Trägermaterial im wesentlichen nicht durchlässig ist, die Bindespezies ein Antikörper ist, der geeignet ist, eine spezifische Bindung mit der Hilfsspezies einzugehen und die Bindespezies geeignet ist, mit Hilfe einer Verknüpfung an die Primärspezies gebunden zu werden, welche eine Bindung eines nicht spezifischen Bindetyps an die Bindespezies beinhaltet, wobei die Bindespezies so angeordnet ist, daß sie mit Hilfe der Verknüpfung an die Primärspezies gebunden wird.

5. Verfahren nach einem der vorhergehenden Ansprüche 1 bis 3,
ferner **dadurch gekennzeichnet, daß** das Verfahren die Verwendung einer Hilfsspezies, welche ein Ligand ist, der auf ein Trägermaterial aufgebracht ist, die Verwendung einer Bindespezies für die Hilfsspezies, wobei die Bindespezies ein Antikörper ist, welcher geeignet ist, eine spezifische Bindung mit der Hilfsspezies einzugehen, und wobei die Bindespezies geeignet ist, mit Hilfe einer Verknüpfung an die Primärspezies gebunden zu werden, welche eine Bindung eines nicht spezifischen Bindungstyps an die Bindespezies beinhaltet, und die Verwendung eines Antikörpers für die zu analysierende Spezies oder die Verwendung eines Antikörpers für eine zu detektierende Substanz beinhaltet.

6. Verfahren nach Anspruch 5,
ferner **dadurch gekennzeichnet, daß** das Verfahren die Verwendung einer Hilfsspezies, welche ein Ligand ist, der auf ein Trägermaterial aufgebracht ist, die Verwendung einer Bindespezies für die Hilfsspezies, wobei die Bindespezies ein Antikörper ist, der geeignet ist, eine spezifische Bindung mit der Hilfsspezies einzugehen, und wobei die Bindespezies eine zu detektierende Substanz trägt, und die Verwendung eines Antikörpers für die zu detektierende Substanz beinhaltet.

7. Verfahren nach Anspruch 4,
ferner **dadurch gekennzeichnet, daß** das Verfahren die Verwendung einer Hilfsspezies, bei der es sich um einen Liganden handelt, der auf ein Trägermaterial aufgebracht ist, die Verwendung einer Bindespezies für die Hilfsspezies, wobei die Bindespezies ein Antikörper ist, der geeignet ist, eine spezifische Bindung mit der Hilfsspezies einzugehen, und wobei die Bindespezies geeignet ist, mit Hilfe einer Verknüpfung an die Primärspezies gebunden zu werden, welche eine Bindung eines nicht spezifischen Bindungstyps an die Bindespezies beinhaltet, und die Verwendung eines Antikörpers für eine zu analysierende Spezies oder die Verwendung eines Antikörpers für eine zu detektierende Substanz beinhaltet.

8. Verfahren nach Anspruch 7,
ferner **dadurch gekennzeichnet, daß** das Verfahren die Verwendung einer Hilfsspezies, bei der es sich um einen Liganden handelt, der auf ein Trägermaterial aufgebracht ist, die Verwendung einer Bindespezies für die Hilfsspezies, wobei die Bindespezies ein Antikörper ist, der geeignet ist, eine spezifische Bindung mit der Hilfsspezies einzugehen, und wobei die Bindespezies eine Substanz trägt, die detektiert werden soll, und die Verwendung eines Antikörpers für die zu detektierende Substanz beinhaltet.

**9.** Verwendung nach einem der vorhergehenden Ansprüche, ferner **dadurch gekennzeichnet, daß** das Trägermaterial oder Teile desselben die Hilfsspezies bereitstellt.

**10.** Verfahren nach einem der Ansprüche 1 bis 8,
ferner **dadurch gekennzeichnet, daß** die Hilfsspezies direkt oder indirekt an das Hilfsmaterial angebracht ist.

**11.** Verfahren nach Anspruch 9 oder Anspruch 10,
ferner **dadurch gekennzeichnet, daß** ein Oligomer einer Hilfsspezies oder ein Polymer einer Hilfsspezies auf dem Trägermaterial bereitgestellt wird.

**12.** Verfahren nach einem der vorhergehenden Ansprüche, ferner **dadurch gekennzeichnet, daß** es sich bei der Hilfsspezies um 2,4-Dinitrophenol, Fluorescein, Digitoxin, Cumarin oder Cibacron-Blau handelt.

**13.** Verfahren nach einem der Ansprüche 1 bis 11,
ferner **dadurch gekennzeichnet, daß** es sich bei der Hilfsspezies um ein Polypeptid, ein Protein, ein Polysaccharid oder ein leitendes Polymer handelt.

**14.** Verfahren nach einem der vorhergehenden Ansprüche, ferner **dadurch gekennzeichnet, daß** die Hilfsspezies als Polymerbeschichtung auf einem Trägermaterial bereitgestellt wird.

**15.** Verfahren nach einem der Ansprüche 1 bis 12 oder Anspruch 14,
ferner **dadurch gekennzeichnet, daß** die Bindespezies ein Anti-Fluorescein-Antikörper, Anti-Cumarin-Antikörper, Anti-2,4-Dinitrophenol-Antikörper oder ein Anti-Cibacron-Blau-Antikörper ist.

**16.** Verfahren nach einem der vorhergehenden Ansprüche, ferner **dadurch gekennzeichnet, daß** die Primärspezies ein Primärantikörper oder ein Ligand ist.

**17.** Verfahren nach einem der vorhergehenden Ansprüche, ferner **dadurch gekennzeichnet, daß** die zu analysierende Spezies als solche detektierbar ist oder die analysierende Spezies Teil einer zu detektierenden Substanz ist.

**18.** Verfahren nach Anspruch 17,
ferner **dadurch gekennzeichnet, daß** die zu analysierende Spezies ein Hormon, eine Droge, ein Steroid, einen Tumor-Marker, menschliches Serumalbumin, menschliches chloriertes Gonadotrophin, menschliches IgG, ein Proteinantigen, ein Blutprotein, ein Markerprotein, ein Giftstoff, ein Mikroorganismus, ein Metallkomplex oder ein Metallion ist.

**19.** Verfahren nach Anspruch 18,
ferner **dadurch gekennzeichnet, daß** es sich bei dem Metallkomplex um Methylquecksilber handelt.

**20.** Verfahren nach Anspruch 18,
ferner **dadurch gekennzeichnet, daß** das Metallion ein Calciumion, ein Eisenion, ein Kobaltion, ein Aluminiumion, ein Zinkion, ein Bleiion, ein Kupferion, ein Cadmiumion, ein Vanadiumion, ein Silberion, ein Quecksilberion, ein Indiumion, ein Manganion oder ein Nickelion ist.

**21.** Verfahren nach einem der vorhergehenden Ansprüche, ferner **dadurch gekennzeichnet, daß** eine zu detektierende Substanz durch Wechselwirkung einer zu analysierenden Spezies mit einem Agens gebildet wird.

**22.** Verfahren nach Anspruch 21,
ferner **dadurch gekennzeichnet, daß** das Agens ein Agens ist, das geeignet ist, einen Komplex zu bilden.

**23.** Verfahren nach Anspruch 21 oder 22,
ferner **dadurch gekennzeichnet, daß** es den Schritt beinhaltet, einen Komplex einer zu analysierenden Spezies zu bilden, wobei der Komplex die zu detektierenden Substanz bereitstellt.

**24.** Verfahren nach einem der Ansprüche 21 bis 23,
ferner **dadurch gekennzeichnet, daß** es die Verwendung eines Agens, wobei das Agens geeignet ist, mit der zu analysierenden Spezies oder mit einer authentischen zu analysierenden Spezies in Wechselwirkung zu treten, um eine zu detektierende Substanz zu bilden, die Verwendung einer Bindespezies, zur Assoziierung mit dem Agens, die Verwendung einer Hilfsspezies, wobei die Bindespezies eine Bindespezies für die Hilfsspezies ist und die Bindespezies und die Hilfsspezies geeignet sind, eine Bindung miteinander einzugehen, die Verwendung eines Trägermaterials und die Verwendung eines Antikörpers für die detektierende Substanz beinhaltet.

25. Verfahren nach einem der Ansprüche 21 bis 24, wobei das Verfahren
ferner **dadurch gekennzeichnet, ist, daß** es die Schritte beinhaltet, eine zu analysierende Spezies und eine Bindespezies zusammenzubringen, welche ein Agens trägt, das geeignet ist, eine Wechselwirkung mit der zu analysierenden Spezies einzugehen und damit eine Wechselwirkung der zu analysierenden Spezies mit dem Agens zu bewirken, um so eine zu detektierende Substanz zu bilden, wobei die Bindespezies, die die gerade gebildete, zu detektierende Substanz trägt, und eine Hilfsspezies, die auf ein Trägermaterial aufgebracht ist, zusammenzubringen, um so eine Bindung zwischen der Bindespezies und der Hilfsspezies zu bewirken, und einen Antikörper zuzugeben, wobei der Antikörper ein Antikörper für die zu detektierende Substanz ist, derart, daß der Antikörper an die zu detektierende Substanz bindet.

26. Verfahren nach einem der Ansprüche 21 bis 24,
ferner **dadurch gekennzeichnet, daß** die Hilfsspezies und eine Bindespezies, die ein Agens trägt, das geeignet ist, eine Wechselwirkung mit der zu analysierenden Spezies einzugehen, so angeordnet sind, daß sie aneinander binden, bevor eine zu analysierende Spezies mit dem Agens in Wechselwirkung tritt, so daß eine zu detektierende Substanz gebildet wird.

27. Verfahren nach einem der Ansprüche 21 bis 26,
ferner **dadurch gekennzeichnet, daß** es die Schritte enthält, eine Bindespezies und eine Hilfsspezies, welche auf ein Trägermaterial aufgebracht ist, zusammenzubringen, um so eine Bindung zwischen der Bindespezies und der Hilfsspezies zu bewirken, die Bindespezies so bereitzustellen, daß sie eine zu detektierende Substanz trägt, einen Antikörper einzubringen, wobei es sich bei dem Antikörper um einen Antikörper für die zu detektierende Substanz handelt und der Antikörper eine Indikatorspezies trägt, derart, daß der Antikörper an die zu detektierende Substanz gebunden wird und die Bindung des Antikörpers und der Substanz mit Hilfe der Indikatorspezies zu detektieren.

28. Verfahren nach einem der Ansprüche 21 bis 27,
ferner **dadurch gekennzeichnet, daß** ein Chelatbildner verwendet wird, um einen Komplex als zu detektierende Substanz zu bilden.

29. Verfahren nach einem der Ansprüche 21 bis 28,
ferner **dadurch gekennzeichnet, daß** es ferner beinhaltet, eine Bindespezies, welche ein oder mehrere Moleküle eines Chelatbildners trägt, und eine zu analysierende Spezies, die Metallionen enthält, zusammenzubringen, um eine Bindespezies zu bilden, die einen Metall-Chelatkomplex als zu detektierende Substanz trägt, die Bindespezies, die den Metall-Chelatkomplex trägt, und eine Hilfsspezies, die auf einem Trägermaterial aufgebracht ist, zusammenzubringen, um so eine Bindung zwischen der Bindespezies und der Hilfsspezies zu bewirken, einen Antikörper einzubringen, wobei der Antikörper ein Antikörper für die zu detektierende Substanz ist und der Antikörper eine detektierbare Spezies trägt, derart, daß der Antikörper an den Metall-Chelatkomplex bindet und die Bindung des Antikörpers und des Metall-Chelatkomplexes zu detektieren.

30. Verfahren nach Anspruch 29, ferner
**dadurch gekennzeichnet, daß** der Antikörper eine detektierbare Spezies trägt, die einen Enzymindikator aufweist und die Bindung des Antikörpers und des Metall-Chelatkomplexes durch einen Enzym-induzierten Übergang detektiert wird.

31. Verfahren nach Anspruch 30, ferner
**dadurch gekennzeichnet, daß** der Enzym-induzierte Übergang ein Farbübergang, ein Fluoreszenzübergang, ein Lumineszenzübergang oder ein elektrochemischer Übergang ist.

32. Verfahren nach Anspruch 28 oder Anspruch 29,
ferner **dadurch gekennzeichnet, daß** der Chelatbildner Ethylendiamintetraacetat, Diethylentriaminpentaacetat, Cyclohexylendinitriltetraessigsäure, 1-Benzyl-ETDA, ein Derivat von 1-Benzyl-EDTA, 8-Hydroxychinolin, ein Derivat von 8-Hydroxychinolin oder Deferoxamin ist.

33. Verfahren nach einem der vorhergehenden Ansprüche, ferner

**dadurch gekennzeichnet, daß** das Trägermaterial ein Material fester Phase und das Material fester Phase eine Wand eines Reaktionsgefäßes ist, ein unlösliches Polysaccharid, ein Microteilchen, Polystyrol, vernetztes Dextran, eine unlösliche Polymerstruktur, eine Glasfläche, eine derivatisierte Siliciumdioxidfläche, ein Polymer, das auf die Fläche aufgebracht ist, ein Material aus Micropartikeln mit eingeschlossenem Eisenoxid, Nylon oder ein Polyamid ist.

34. Verfahren nach einem der Ansprüche 1 bis 28 oder 32 oder 33, ferner
**dadurch gekennzeichnet, daß** eine detektierbare Spezies verwendet wird.

35. Verfahren nach Anspruch 34, ferner
**dadurch gekennzeichnet, daß** eine detektierbare Spezies verwendet wird und die detektierbare Spezies ein Enzym, ein Fluorophor, ein polymeres Fluorophor, ein Radioisotop, ein Ligand, ein Polymer eines Liganden oder ein Bindemittel aufweist.

36. Verfahren nach Anspruch 35, ferner
**dadurch gekennzeichnet, daß** eine detektierbare Spezies ein Ligand ist und der Ligand über die Verwendung eines Bindemittels für diesen detektiert wird oder die detektierbare Spezies ein Bindemittel ist und das Bindemittel unter Verwendung eines Liganden für dieses detektiert wird.

37. Verfahren nach einem der vorhergehenden Ansprüche, ferner
**dadurch gekennzeichnet,**
**daß** eine Indikatorspezies verwendet wird.

38. Verfahren nach Anspruch 37, ferner
**dadurch gekennzeichnet,**
**daß** es sich bei der Indikatorspezies um ein Enzym, ein Fluorophor, eine chemilumineszierende Verbindung, eine biolumineszierende Verbindung, ein Radioisotop oder einen Farbstoff handelt.

39. Verfahren nach Anspruch 38, ferner
**dadurch gekennzeichnet,**
**daß** die Indikatorspezies alkalische Phosphatase, β-Galactosidase, Meerrettich-Peroxidase, Fluorescein, Cumarin oder Rhodamin ist.

40. Verfahren nach einem der Ansprüche 1 bis 8 oder 10 bis 39, ferner
**dadurch gekennzeichnet,**
**daß** es beinhaltet, eine Hilfsspezies auf einem Trägermaterial aufzubringen.

41. Verfahren nach einem der Ansprüche 1 bis 8 oder 10 bis 40, ferner
**dadurch gekennzeichnet,**
**daß** es den Schritt beinhaltet, ein Agens an eine Bindespezies entweder direkt oder indirekt zu binden.

42. Verfahren nach einem der vorhergehenden Ansprüche, ferner
**dadurch gekennzeichnet,**
**daß** eine Probe Wasser, Erde, einer lebendigen Spezies oder Luft eine zu analysierende Spezies oder eine zu detektierende Substanz zur Detektion bereitstellt.

43. Verfahren nach einem der vorhergehenden Ansprüche, ferner
**dadurch gekennzeichnet,**
**daß** eine biologische Probe einer Detektion unterworfen wird.

44. Verfahren nach Anspruch 43, ferner
**dadurch gekennzeichnet,**
**daß** die biologische Probe Blut, Plasma, Serum, Urin, Speichel oder Milch ist.

45. Verfahren nach einem der vorhergehenden Ansprüche, ferner
**dadurch gekennzeichnet,**
**daß** eine zu analysierende Spezies oder eine zu detektierende Substanz in Wasser, einer wäßrigen Zubereitung oder einem Fluidextrakt vorliegt.

**46.** Kompetitives Immunoassay-Verfahren, wobei das Verfahren
**dadurch gekennzeichnet**
**ist, daß** es die Verwendung eines Trennverfahrens nach Anspruch 1 umfaßt.

**47.** Nicht kompetitives Immunoassay-Verfahren, wobei das Verfahren
**dadurch gekennzeichnet**
**ist, daß** es die Verwendung eines Trennverfahrens nach Anspruch 1 beinhaltet.

**48.** Verwendung eines Sensors,
**dadurch gekennzeichnet,**
**daß** gemäß einem Verfahren nach einem der Ansprüche 4 bis 47 der Sensor ein Trägermaterial beinhaltet und eine Hilfsspezies auf dem Trägermaterial vorgesehen ist.

**49.** Verwendung eines Trägermaterials,
**dadurch gekennzeichnet,**
**daß** in einem Trennverfahren nach einem der Ansprüche 1 bis 3 oder der Ansprüche 5 bis 47 eine Hilfsspezies auf dem Trägermaterial vorgesehen ist.

**50.** Verwendung eines Trägermaterials,
**dadurch gekennzeichnet,**
**daß** in einem Verfahren nach einem der Ansprüche 4 bis 47 eine Hilfsspezies auf dem Trägermaterial vorgesehen ist.

**51.** Verwendung eines Test-Kits,
**dadurch gekennzeichnet,**
**daß** in einem Verfahren nach einem der Ansprüche 4 bis 47 das Test-Kit ein Trägermaterial und eine Hilfsspezies beinhaltet, welche auf dem Trägermaterial vorgesehen ist.

## Revendications

**1.** Procédé de séparation, adapté pour être utilisé dans un procédé d'essai immunologique pour la détection d'un analyte, lequel procédé de séparation est **caractérisé en ce que** ledit procédé de séparation comporte la possibilité qu'une espèce auxiliaire et qu'une espèce de fixation pour l'espèce auxiliaire subissent une fixation spécifique, ladite espèce auxiliaire n'étant pas une espèce primaire et ladite espèce auxiliaire étant un ligand et ladite espèce auxiliaire étant agencée sur un matériau de support, ledit matériau de support étant essentiellement non-perméable, ladite espèce de fixation étant un anticorps capable d'une fixation spécifique avec l'espèce auxiliaire et ladite espèce de fixation étant capable d'être liée à une espèce primaire par l'intermédiaire d'une liaison qui implique un lien, d'un type de fixation non-spécifique, à l'espèce de fixation, ladite espèce de fixation étant conçue pour être reliée à une espèce primaire par l'intermédiaire de ladite liaison.

**2.** Procédé selon la revendication 1, caractérisé de plus en ce que le lien d'un type de fixation non-spécifique est un lien covalent ou un lien qui implique une adsorption.

**3.** Procédé selon la revendication 1 ou 2, caractérisé de plus en ce que la liaison comporte également un autre lien d'un type de fixation non-spécifique ou un autre lien d'un type de fixation spécifique.

**4.** Procédé adapté pour être utilisé dans la détection d'un analyte par un essai immunologique, lequel procédé est **caractérisé en ce que** ledit procédé inclut la possibilité qu'une espèce auxiliaire et qu'une espèce de fixation pour l'espèce auxiliaire subissent une fixation spécifique, ladite espèce auxiliaire n'étant pas une espèce primaire et ladite espèce auxiliaire étant un ligand et ladite espèce auxiliaire étant agencée sur un matériau de support, ledit matériau de support étant essentiellement non-perméable, ladite espèce de fixation étant un anticosps capable d'une fixation spécifique avec l'espèce auxiliaire et ladite espèce de fixation étant capable d'être reliée à une espèce primaire par l'intermédiaire d'une liaison qui implique un lien, d'un type de fixation non-spécifique, à l'espèce de fixation, ladite espèce de fixation étant conçue pour être reliée à une espèce primaire par l'intermédiaire de ladite liaison.

**5.** Procédé selon l'une quelconque des revendications 1 à 3, caractérisé de plus en ce que le procédé comporte l'utilisation d'une espèce auxiliaire, qui est un ligand, agencée sur un matériau de support, l'utilisation d'une espèce de fixation pour l'espèce auxiliaire, ladite espèce de fixation étant un anticorps capable de se fixer de manière spécifique à l'espèce auxiliaire et ladite espèce de fixation étant capable d'être reliée à une espèce primaire par l'intermédiaire d'une liaison qui implique un lien, d'un type de fixation non-spécifique, à l'espèce de fixation, et l'utilisation d'un anticorps de l'analyte ou l'utilisation d'un anticorps d'une entité à détecter.

6. Procédé selon la revendication 5, caractérisé de plus en ce que le procédé comporte l'utilisation d'une espèce auxiliaire, qui est un ligand, agencée sur un matériau de support, l'utilisation d'une espèce de fixation pour l'espèce auxiliaire, ladite espèce de fixation étant un anticorps capable de se fixer de manière spéaifique à l'espèce auxiliaire et ladite espèce de fixation portant une entité à détecter, et l'utilisation d'un anticorps de l'entité à détecter.

7. Procédé selon la revendication 4, caractérisé de plus en ce que le procédé comporte l'utilisation d'une espèce auxiliaire, qui est un ligand, agencée sur un matériau de support, l'utilisation d'une espèce de fixation pour l'espèce auxiliaire, ladite espèce de fixation étant un anticorps capable d'une fixation spécifique avec l'espèce auxiliaire et ladite espèce de fixation étant capable d'être reliée à une espèce primaire par l'intermédiaire d'une liaison qui implique un lien, d'un type de fixation non-spécifique, à l'espèce de fixation, et l'utilisation d'un anticorps d'un analyte ou l'utilisation d'un anticorps d'une entité à détecter.

8. Procédé selon la revendieation 7, caractérisé de plus en ce que le procédé comporte l'utilisation d'une espèce auxiliaire, qui est un ligand, agencée sur un matériau de support, l'utilisation d'une espèce de fixation pour l'espèce auxiliaire, ladite espèce de fixation étant un anticorps capable d'une fixation spécifique avec l'espèce auxiliaire et ladite espèce de fixation portant une entité à détecter, et l'utilisation d'un anticorps de l'entité à détecter.

9. Procédé selon l'une quelconque des revendications précédentes, caractérisé de plus en ce que le matériau de support, ou une partie de celui-ci, fournit l'espèce auxiliaire.

10. Procédé selon l'une quelconque des revendications 1 à 8, caractérisé de plus en ce que l'espèce auxiliaire est attachée directement, ou indirectement, au matériau de support.

11. Procédé selon la revendication 9 ou la revendication 10, caractérisé de plus en ce qu'un oligomère d'une espèce auxiliaire ou un polymère d'une espèce auxiliaire est agencé sur le matériau de support.

12. Procédé selon l'une quelconque des revendications précédentes, caractérisé de plus en ce que l'espèce auxiliaire est un 2,4 dinitrophénol, une fluorescéine, une digitoxine, une coumarine ou un bleu cibacron.

13. Pxocédé selon l'une quelconque des revendications 1 à 11, caractérisé de plus en ce que l'espèce auxiliaire est un polypeptide, une protéine, un polysaccharide ou un polymère conducteur.

14. Procédé selon l'une quelconque des revendications précédentes, caractérisé de plus en ce que l'espèce auxiliaire est fournie sous forme d'un revêtement d'un polymère sur un matériau de support.

15. Procédé selon l'une quelconque des revendications 1 à 12 ou la revendication 14, caractérisé de plus en ce que l'espèce de fixation est un anticorps anti-fluorescéine, un anticorps anti-coumarine, un anticorps anti-2,4 dinitrophénol ou un anticorps anti-bleu cibacron.

16. Procédé selon l'une quelconque des revendications précédentes, caractérisé de plus en ce que l'espèce primaire est un anticorps primaire ou un ligand.

17. Procédé selon l'une quelconque des revendications précédentes, caractérisé de plus en ce que l'analyte est détectable tel quel ou dans lequel l'analyte est une partie d'une entité à détecter.

18. Procédé selon la revendication 17, caractérisé de plus en ce que l'analyte est une hormone, un médicament, un stéroïde, un marqueur tumoral, une sérumalbumine humaine, une gonadotrophine chlorionique humaine, une IgG humaine, un antigène protéinique, une protéine sanguine, une protéine de marquage, une toxine, un micro-organisme, un complexe métallique ou un ion métallique.

19. Procédé selon la revendication 18, caractérisé de plus en ce que le complexe métallique est un mercure méthylique.

**20.** Procédé selon la revendication 18, caractérisé de plus en ce que l'ion métallique est un ion calcium, un ion fer, un ion cobalt, un ion aluminium, un ion zinc, un ion plomb, un ion cuivre, un ion cadmium, un ion vanadium, un ion argent, un ion mercure, un ion indium, un ion manganèse ou un ion nickel.

**21.** Procédé selon l'une quelconque des revendications précédentes, caractérisé de plus en ce qu'une entité à détecter est formée par interaction d'un analyte avec un agent.

**22.** Procédé selon la revendication 21, caractérisé de plus en ce que l'agent est un agent capable de former un complexe.

**23.** Procédé selon la revendication 21 ou 22, caractérisé de plus en ce qu'il comporte l'étape consistant à former un complexe d'un analyte, ledit complexe fournissant une entité à détecter.

**24.** Procédé selon l'une quelconque des revendications 21 à 23, caractérisé de plus en ce qu'il comporte l'utilisation d'un agent, ledit agent étant capable d'interagir avec un analyte, ou avec un analyte authentique, pour former une entité à détecter, l'utilisation d'une espèce de fixation pour l'association avec l'agent, l'utilisation d'une espèce auxiliaire, ladite espèce de fixation étant une espéae de fixation pour l'espèce auxiliaire et ladite espèce de fixation et ladite espèce auxiliaire étant telles quelles sont capables de se fixer ensemble, l'utilisation d'un matériau de support, et l'utilisation d'un anticorps de l'entité à détecter.

**25.** Procédé selon l'une quelconque des revendications 21 à 24, lequel procédé est de plus **caractérisé en ce qu'**il inclut les étapes consistant à mettre ensemble un analyte et une espèce de fixation, à porter un agent capable d'nne interaction avec l'analyte, de manière à effectuer une interaction de l'analyte avec l'agent de manière à former une entité à détecter, à mettre ensemble l'espèce de fixation, à porter l'entité à détecter ainsi formée, et une espèce auxiliaire agencée sur un matériau de support, de manière à provoquer la fixation entre l'espèce de fixation et l'espèce auxiliaire, et à appliquer un anticorps, ledit anticorps étant un anticorps de l'entité à détecter, de sorte que l'anticorps se fixe à l'entité à détecter.

**26.** Procédé selon l'une quelconque des revendications 21 à 24, caractérisé de plus en ce que l'espèce auxiliaire et une espèce de fixation, portant un agent capable d'une interaction avec un analyte, sont conçues pour se fixer ensemble avant qu'un analyte interagisse avec l'agent pour former une entité à détecter.

**27.** Procédé selon l'une quelconque des revendications 21 à 26, caractérisé de plus en ce qu'il comporte les étapes consistant à mettre ensemble les espèces de fixation et une espèce auxiliaire agencée sur un matériau de support, de manière à provoquer la fixation entre l'espèce de fixation et l'espèce auxiliaire, à s'arranger pour que l'espèce de fixation porte une entité à détecter, à appliquer une anticorps, ledit anticorps étant un anticorps de l'entité à détecter et ledit anticorps portant un marqueur, de sorte que l'anticorps se fixe à l'entité à détecter et à détecter, par l'intermédiaire du marqueur, la fixation de l'anticorps et de l'entité.

**28.** Procédé selon l'une quelconque des revendications 21 à 27, caractérisé de plus en ce qu'un agent chélatant est utilisé pour former un complexe en tant qu'entité à détecter.

**29.** Procédé selon l'une quelconque des revendications 21 à 28, caractérisé de plus en ce qu'il comporte les étapes consistant à mettre ensemble une espèce de fixation, à porter une ou plusieurs molécules d'un agent chélatant, et un analyte comportant des ions métalliques pour former une espèce de fixation portant un complexe métal-chélatant en tant qu'entité à détecter, à mettre ensemble l'espèce de fixation, portant le complexe métal-chélatant, une espèce auxiliaire agencée sur un matériau de support, de manière à provoquer la fixation entre l'espèce de fixation et l'espèce auxiliaire, à appliquer un anticorps, ledit anticorps étant un anticorps de l'entité à détecter et ledit anticorps portant une espèce délectable, de sorte que l'anticorps se fixe au complexe métal-chélatant et à détecter la fixation de l'anticorps et du complexe métal-chélatant.

**30.** Procédé selon la revendication 29, caractérisé de plus en ce que l'anticorps porte une espèce détectable comportant un marqueur enzymatique et la fixation de l'anticorps et du complexe métal-chélatant est détectée par un changement induit par enzyme.

**31.** Procédé selon la revendication 30, caractérisé de plus en ce que le changement induit par enzyme est un changement de couleur, un changement de fluorescence, un changement de luminescence ou un changement électrochimique.

**32.** Procédé selon la revendication 28 ou 29, caractérisé de plus en ce que l'agent chélatant est du tétraacétate d'éthylènediamine, de pentaacétate de diéthylènetriamine, de l'acide tétraaéétique cyclohexylènedinitrilo, du 1-benzyle-EDTA, un dérivé de 1-benzyle-EDTA, la 8-hydroxyquinoline, un dérivé de 8-hydroxyquinoline, ou une deferoxamine.

**33.** Procédé selon l'une quelconque des revendications précédentes, caractérisé de plus en ce que le matériau de support est un matériau en phase solide et le matériau en phase solide est une paroi de récipient de réaction, un polysaccharide insoluble, une microparticule, du polystyrène, du dextran réticulé, une structure polymère insoluble, une surface de verre, une surface de silice modifiée, un polymère attaché à une surface, un matériau microparticulaire avec de l'oxyde ferreux piégé, du Nylon ou un polyamide.

**34.** Procédé selon l'une quelconque des revendications 1 à 28, ou 32, ou 33, caractérisé de plus en ce qu'une espèce détectable est utilisée.

**35.** Procédé selon la revendication 34, caractérisé de plus en ce qu'une espèce détectable est utilisée et l'espèce détectable comporte une enzyme, un fluorophore, un fluorophore polymère, un radio-isotope, un ligand, un polymère d'un ligand, ou un liant.

**36.** Procédé selon la revendication 35, caractérisé de plus en ce que l'espèce détectable est un ligand et le ligand est détecté en utilisant un liant de celui-ci, ou l'espèce détectable est un liant et le liant est détecté par l'utilisation d'un ligand de celui-ci.

**37.** Procédé selon l'une quelconque des revendications précédentes, caractérisé de plus en ce qu'un marqueur est utilisé.

**38.** Procédé selon la revendication 37, caractérisé de plus en ce que le marqueur est une enzyme, un fluorophore, un composé chimioluminescent, un composé bioluminescent, un radio-isotope ou un colorant.

**39.** Procédé selon la revendication 38, caractérisé de plus en ce que le marqueur est une phosphatase alcaline, une β-galactosidase, une peroxydase du raifort, un fluorescéine, une coumarine, ou une rhodamine.

**40.** Procédé selon l'une quelconque des revendications 1 à 8 ou 10 à 39, caractérisé de plus en ce qu'il comporte la fixation d'une espèce auxiliaire à un matériau de support.

**41.** Procédé selon l'une quelconque des revendications 1 à 8, ou 10 à 40, caractérisé de plus en ce qu'il comporte l'étape consistant à relier, directement ou indirectement, un agent à une espèce de fixation.

**42.** Procédé selon l'une quelconque des revendications précédentes, caractérisé de plus en ce qu'un échantillon d'eau, de terre, d'une espèce vivante, ou d'air fournit un analyte, ou une entité à détecter, pour une détection.

**43.** Procédé selon l'une quelconque des revendications précédentes, caractérise de plus en ce qu'un échantillon biologique est soumis à une détection.

**44.** Procédé selon la revendication 43, caractérisé de plus en ce que l'échantillon biologique est du sang, du plasma, du sérum, de l'urine, de la salive ou du lait.

**45.** Procédé selon l'une quelconque des revendications précédentes, caractérisé de plus en ce qu'un analyte, ou une entité à détecter, est présent dans l'eau, une préparation aqueuse, ou un extrait de fluide.

**46.** Procédé d'essai immunologique compétitif, lequel procédé est **caractérisé en ce qu'**il inclut l'utilisation d'un procédé de séparation selon la revendication 1.

**47.** Procédé d'essai immunologique non-compétitif, lequel procédé est **caractérisé en ce qu'** il inclut l'utilisation d'un procédé de séparation selon la revendication 1.

**48.** Utilisation d'un capteur, **caractérisée en ce que** ledit capteur comporte un matériau de support et une espèce auxiliaire agencée sur ledit matériau de support, dans un procédé selon l'une quelconque des revendications 4 à 47.

**49.** Utilisation d'un matériau de support, **caractérisée en ce qu'**une espèce auxiliaire est agencée sur ledit matériau de support, dans un procédé de séparation selon l'une quelconque des revendications 1 à 3 ou 5 à 47.

**50.** Utilisation d'un matériau de support, **caractérisée en ce qu'**une espèce auxiliaire est agencée sur ledit matériau de support, dans un procédé selon l'une quelconque des revendications 4 à 47.

**51.** Utilisation d'un kit de test, **caractérisée en ce que** ledit kit de test inclut un matériau de support et une espèce auxiliaire agencée sur ledit matériau de support, dans un procédé selon l'une quelconque des revendications 4 à 47.